(19) Europäisches Patentamt
European Patent Office
Office européen des brevets

(11) **EP 0 950 043 B1**

(12) # FASCICULE DE BREVET EUROPEEN

(45) Date de publication et mention de la délivrance du brevet:
**07.11.2001 Bulletin 2001/45**

(21) Numéro de dépôt: **97952974.0**

(22) Date de dépôt: **22.12.1997**

(51) Int Cl.$^7$: **C07C 43/178**, C07C 43/196, C08G 65/26, C11D 1/722

(86) Numéro de dépôt international:
**PCT/FR97/02381**

(87) Numéro de publication internationale:
**WO 98/28249 (02.07.1998 Gazette 1998/26)**

(54) **COMPOSES TERPENIQUES POLYALCOXYLES, LEUR PROCEDE DE PREPARATION ET LEUR UTILISATION COMME AGENTS DEMOUSSANTS**

POLYALKOXYTERPENOIDE, VERFAHREN ZU IHRER HERSTELLUNG UND IHRE VERWENDUNG ALS ENTSCHAUMUNGSMITTEL

POLYALCOXYLATED TERPENIC COMPOUNDS, METHOD OF PREPARATION AND USE AS ANTIFOAMING AGENTS

(84) Etats contractants désignés:
**AT BE CH DE DK ES FI FR GB GR IE IT LI LU NL PT SE**

(30) Priorité: **20.12.1996 FR 9615712**

(43) Date de publication de la demande:
**20.10.1999 Bulletin 1999/42**

(73) Titulaire: **RHODIA CHIMIE**
**92408 Courbevoie Cédex (FR)**

(72) Inventeurs:
- **JOYE, Jean-Luc**
  **F-75009 Paris (FR)**
- **FROUTE, Agnès**
  **Cheshire WA15 0DE (GB)**

(74) Mandataire: **Delenne, Marc et al**
**Rhodia Services,**
**Direction de la Propriété Industrielle,**
**40, rue de la Haie-Coq**
**93306 Aubervilliers Cedex (FR)**

(56) Documents cités:
**WO-A-96/01245** **US-A- 3 370 080**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention a pour objet de nouveaux composés terpéniques polyalcoxylés, leur procédé de préparation et leur utilisation comme agents démoussants de milieux aqueux moussants, le terme "terpénique" signifiant "d'origine terpénique".

Il est connu, d'après la demande internationale WO 96/01245 que des dérivés terpéniques bicycloheptanes ou bicycloheptènes polyalcoxylés, dont le groupement polyalcoxy comprend une séquence polyoxyéthylène et/ou une séquence polyoxypropylène, sont des agents tensioactifs peu moussants.

Il a maintenant été trouvé que des dérivés terpéniques bicycloheptanes ou bicycloheptènes polyalcoxylés dont le groupement polyalcoxy comprend au moins une séquence polyoxyéthylène et au moins deux séquences polyoxyalkylènes supérieures (séquences polyoxypropylènes notamment), l'une desdites séquences polyoxyalkylènes supérieures se trouvant en extrémité de chaîne desdits dérivés terpéniques polyalcoxylés, sont non seulement peu moussants mais présentent en outre des propriétés performantes de démoussage des milieux aqueux moussants.

Un premier objet de l'invention consiste en de nouveaux composés terpéniques polyalcoxylés de formule (I)

$$Z - X - W - [CH(R^5)-CH(R^6)-O]_q - A \qquad (I)$$

formule dans laquelle :

- le symbole Z représente un radical bicyclo[a,b,c]hepténylе ou bicyclo[a,b,c]heptyle, éventuellement substitué par au moins un radical alkyle en $C_1$-$C_6$, méthyle de préférence,
  a, b et c étant tels que ;
  * a + b + c = 5,
  * a = 2, 3 ou 4
  * b = 1 ou 2
  * c = O ou 1
- le symbole X représente un groupement
  * $-CH_2-C(R^1)(R^2)-O-$
  * ou $-O-CH(R'^1)-CH(R'^2)-O-$

  les symboles $R^1$ et $R^2$ étant identiques ou différents et représentant un groupement (cyclo)alkyle ou (cyclo)alcényle linéaire ou ramifié en $C_1$-$C_6$, méthyle notamment, ou de préférence un atome d'hydrogène les symboles $R'^1$ et $R'^2$ étant identiques ou différents et représentant un groupement (cyclo)alkyle ou (cyclo)alcényle linéaire ou ramifié en $C_1$-$C_{22}$, méthyle notamment, ou de préférence un atome d'hydrogène,
- les symboles $R^5$ et $R^6$ sont différents, l'un représentant un atome d'hydrogène et l'autre un groupement (cyclo) alkyle ou (cyclo)alcényle linéaire ou ramifié en $C_1$-$C_{22}$, méthyle de préférence,
- q est une valeur moyenne pouvant aller de 1 à 30, de préférence de 5 à 20,
- W représente un groupement polyséquencé constitué de séquences différentes - $[B]_n$ - et - $[C]_p$-,
  . B représentant un groupement $-CH(R^3)-CH(R^4)-O-$ , dans lequel $R^3$ et $R^4$ sont identiques ou différents et représentent un atome d'hydrogène ou un groupe en $C_1$-$C_{22}$ (cyclo)alkyle ou (cyclo)alcényle linéaire ou ramifié, de préférence méthyle, $R^3$ et $R^4$ étant différents lorsque l'un d'eux représente de l'hydrogène
  . C représentant un groupement oxyéthyléné $-CH_2-CH_2-O-$ (EO)
  . n étant une valeur moyenne pouvant aller de 1 à 10, de préférence de 2 à 4
  . p étant une valeur moyenne pouvant aller de 1 à 100, de préférence de 3 à 20 ledit groupement polyséquencé W étant lié au motif X par une de ses séquences - $[B]_n$-- A représente un atome d'hydrogène, un radical alkyle en $C_1$-$C_6$, un radical aryle ou alkylaryle, un atome d'halogène, un groupement $-CH_2-CH(OH)R^7$, où le symbole $R^7$ représente un radical alkyle linéaire ou ramifié ou cyclique en $C_1$-$C_{22}$ ou aryle, ou un groupement choisi parmi $-SO_3M$, $-OPO_3(M)_2$, $-(CH_2)_a-COOM$, $-(CH_2)_b-SO_3M$, avec a et b allant de 1 à 6, M représentant H , Na , K , Li , $N(RR'R''R''')^+$ où les symboles R, R', R'' et R''' sont identiques ou différents et représentent un atome d'hydrogène ou un radical alkyle linéaire ou ramifié ou cyclique en $C_1$-$C_{22}$ éventuellement hydroxylé.

Une représentation des squelettes de Z bicyclo[a,b,c]hepténique non substitué et bicyclo[a,b,c]heptanique

non substitué est donnée aux figures 1 et 2.
D'une manière préférentielle, le symbole Z représente un radical $Z^1$ ou $Z^2$,

* $Z^1$ étant un radical
  - bicyclo[3.1.1]heptényle, de préférence substitué sur son atome de carbone en 6 par au moins un radical alkyle en $C_1$-$C_6$, tout particulièrement par deux radicaux méthyles, ledit radical bicyclo[3.1.1]heptényle étant lié au motif X de formule $-CH_2-C(R^1)(R^2)-O-$, par l'intermédiaire de son atome de carbone en 2 ;
  - ou bicyclo[2.2.1]heptényle, de préférence substitué sur son atome de carbone en 7 par au moins un radical alkyle en $C_1$-$C_6$, tout particulièrement par deux radicaux méthyles, ledit radical bicyclo[2.2.1]heptényle étant lié au motif X de formule $-CH_2-C(R^1)(R^2)-O-$, par l'intermédiaire de son atome de carbone en 2 ou en 3 ;

* $Z^2$ étant un radical bicyclo[2.2.1]heptyle, de préférence substitué sur son atome de carbone en 7 par au moins un radical alkyle en $C_1$-$C_6$, tout particulièrement par deux radicaux méthyles.
  ledit radical bicyclo[2.2.1]heptyle étant lié au motif X de formule $-O-CH(R'^1)-CH(R'^2)-O-$, par l'intermédiaire de son atome de carbone en 2 ou en 3.

D'une manière préférentielle, les dites séquences $[B]_n$ et $[CH(R^5)-CH(R^6)-O]_q$ sont des séquences polyoxypropylénées $[PO]_n$ et $[PO]_q$.
D'une manière tout particulièrement préférentielle, le symbole W représente un groupement biséquencé $-[B]_n-[C]_p-$ , la séquence $[B]_n$ étant une séquence polyoxypropylénée $[PO]_n$ et la séquence $[C]_p$ étant une séquence polyoxyéthylénée $[EO]_p$.
La présente invention a plus particulièrement pour objet les nouveaux composés terpéniques polyalcoxylés de formule

$$Z^1 - CH_2-CH_2- O - [PO]_n-[EO]_p- [PO]_q- A$$

ou

$$Z^2 - O-CH_2-CH_2- O - [PO]_n-[EO]_p- [PO]_q- A$$

$Z^1$ et $Z^2$ ayant la définition donnée ci-dessus.
A titre d'exemples de ces nouveaux composés on peut mentionner notamment ceux de formule

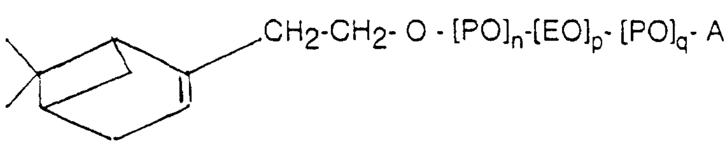

ou

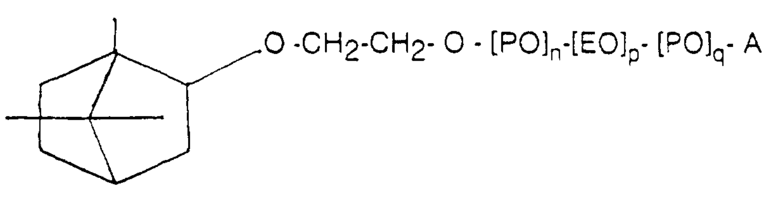

D'une manière préférentielle, les valeurs moyennes de n, p et q sont choisies de façon à ce qu'une solution à 1% en poids dans l'eau distillée dudit composé présente un point de trouble inférieur à 40°C.
Les composés faisant l'objet de l'invention, peuvent être préparés par réactions de polyalcoxylations successives du réactif de formule Z - XH, par au moins deux types d'agents d'alcoxylation différents, dont l'un est de l'oxyde d'éthylène et le ou les autres un oxyde d'alkylène supérieur, avec alcoxylation finale à l'aide d'un oxyde d'alkylène supérieur, puis fonctionnalisation éventuelle de l'atome d'hydrogène terminal.

Plus précisément ledit réactif Z - XH est soumis à des réactions de polyalcoxylations successives, avec de l'oxyde d'alkylène (OA1) de formule

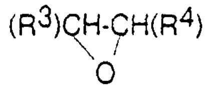

de l'oxyde d'alkylène (OE) de formule

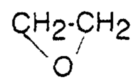

et de l'oxyde d'alkylène (OA2) de formule

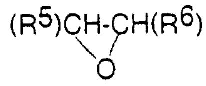

$R^3$, $R^4$, $R^5$ et $R^6$ ayant la définition donnée ci-dessus,
avec introduction successive des oxydes d'alkylènes (OA1) et (OE) et introduction finale d'oxyde d'alkylène (OA2), pour obtenir un produit de formule

$$Z - X - W - [CH(R^5)-CH(R^6)-O]_q H$$

dans laquelle W et q ont la définition donnée ci-dessus,
puis éventuellement fonctionnalisation pour transformer l'atome d'hydrogène terminal en un des substituants A autres que l'hydrogène tels que définis ci-dessus.

Ainsi les nouveaux composés terpéniques polyalcoxylés de formule (I) faisant l'objet de l'invention, formule dans laquelle X représente $-CH_2-C(R^1)(R^2)-O-$, peuvent être obtenus par réaction de polyalcoxylation d'un réactif de formule (I')

$$Z - CH_2-C(R^1)(R^2)\ OH \qquad (I')$$

dans laquelle les symboles Z, $R^1$ et $R^2$ ont la définition donnée ci-dessus,
avec de l'oxyde d'alkylène (OA1) de formule

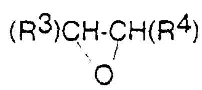

de l'oxyde d'alkylène (OE) de formule

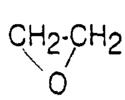

et de l'oxyde d'alkylène (OA2) de formule

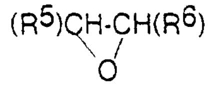

$R^3$, $R^4$, $R^5$ et $R^6$ ayant la définition donnée ci-dessus,
avec introduction successive des oxydes d'alkylènes (OA1) et (OE) et introduction finale d'oxyde d'alkylène (OA2), pour obtenir un produit de formule

$$Z - CH_2\text{-}C(R^1)(R^2)\text{-}O\text{- } W \text{ - } [CH(R^5)\text{-}CH(R^6)\text{-}O]_q \text{ } H$$

dans laquelle W et q ont la définition donnée ci-dessus,
puis éventuellement fonctionnalisation pour transformer l'atome d'hydrogène terminal en un des substituants A autres que l'hydrogène tels que définis ci-dessus.

Les nouveaux composés terpéniques polyalcoxylés de formule (I) faisant l'objet de l'invention, formule dans laquelle X représente -O-CH(R'1)-CH(R'2)-O-, peuvent être obtenus par réaction de polyalcoxylation d'un réactif de formule (I")

$$Z\text{ -O-CH}(R'^1)\text{-CH}(R'^2)\text{-OH} \qquad (I")$$

dans laquelle les symboles Z, $R'^1$ et $R'^2$ ont la définition donnée ci-dessus,
avec de l'oxyde d'alkylène (OA1) de formule

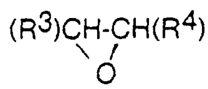

de l'oxyde d'alkylène (OE) de formule

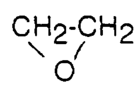

et de l'oxyde d'alkylène (OA2) de formule

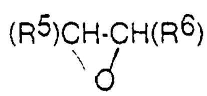

$R^3$, $R^4$, $R^5$ et $R^6$ ayant la définition donnée ci-dessus,
avec introduction successive des oxydes d'alkylènes (OA1) et (OE) et introduction finale d'oxyde d'alkylène (OA2), pour obtenir un produit de formule

$$Z \text{ - O-CH}(R'^1)\text{-CH}(R'^2)\text{-O- } W \text{ - } [CH(R^5)\text{-}CH(R^6)\text{-}O]_q \text{ } H$$

dans laquelle W et q ont la définition donnée ci-dessus,
puis éventuellement fonctionnalisation pour transformer l'atome d'hydrogène terminal en un des substituants A autres que l'hydrogène tels que définis ci-dessus.

Les modes de préparation des réactifs de formule (I') et (I") sont décrits dans la demande internationale WO 96/01245.

D'une manière tout particulièrement préférentielle les réactifs de formule (I') et (I") ont pour formule (II') et (II")

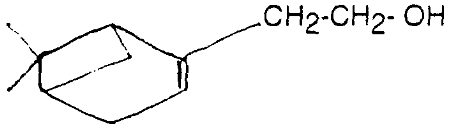

(II')

dénommé "NOPOL", obtenu par réaction du béta-pinène avec du formaldéhyde ;

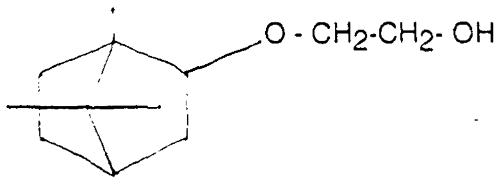

(II")

dénommé "ARBANOL", obtenu par isomérisation de l'alpha-pinène en camphène puis éthoxyhydroxylation.

D'une manière préférentielle, les oxydes d'alkylènes (OA1) et (OA2) sont de l'oxyde de propylène.

Les réactions de polyalcoxylation sont réalisées selon les méthodes bien connues à une température supérieure à 100°C, de préférence entre 120 et 250°C, tout particulièrement entre 150 à 200°C, en présence d'un catalyseur (bases fortes, amines aliphatiques, acides de Lewis). D'une manière avantageuse, l'opération est réalisée en présence d'un gaz inerte (azote) ou d'un gaz rare (argon ou de monoxyde de carbone, de préférence à une pression de l'ordre de 1 à 4 bar. De plus amples détails concernant ce type de réaction sont donnés dans la demande internationale WO 96/01245.

L'oxyde d'alkylène (OA1) et l'oxyde d'éthylène (OE) sont introduits successivement, avant l'introduction finale de l'oxyde d'alkylène (OA2).
Les quantités d'oxyde d'alkylène ou d'éthylène mises en oeuvre correspondent aux nombres d'équivalents molaires n, p et q recherchés.Les conditions de réalisation d'un tel mode opératoire sont bien connues de l'homme de l'art.

L'opération de fonctionnalisation éventuelle pour transformer l'atome d'hydrogène terminal en un des subsituants A autres, peut être, par exemple, une opération d'éthérification ou d'estérification de l'atome d'hydrogène terminal ; cette étape est bien connue en elle-même ; elle est de préférence réalisée après neutralisation.
Ainsi, peut être réalisée la préparation

- d'éthersulfates (A = $-SO_3M$ ), selon le mode opératoire décrit dans GB 1 111 208 ou US-A-3,392,185
- d'étherphosphates (A = $-OPO_3(M)_2$ ), selon le mode opératoire décrit dans US-A-3,331,896
- d'éthercarboxylates (A = $-(CH_2)_a-COOM$ ), selon le mode opératoire décrit dans US-A-2,623,900 ou US-A-2,983,738
- d'éthersulfonates (A = $-(CH_2)_b-SO_3M$ ), selon le mode opératoire décrit dans US-A-2,115,192 , US-A-4,978,780 ou K. SUGA, Austr. J. Chem., 21, 2333 (1968)
- d'alkyléthers (A = alkyle ), selon le mode opératoire décrit dans US-A-2,913,416.

De plus amples détails concernant ces modes de fonctionnalisation sont décrits dans WO 96/01245.

La présente invention a également pour objet l'utilisation comme agent démoussant dans des milieux aqueux susceptibles de former des mousses, d'au moins un composé terpénique polyalcoxylé de formule (I) tel que défini ci-dessus.

Les milieux aqueux, dont il faut limiter le volume de mousse susceptible de se former lors de leur utilisation, sont notamment les milieux aqueux de dégraissage en milieu alcalin des tôles métalliques, les milieux aqueux de dégraissage des plates-formes de forage et ceux des milieux aqueux mis en oeuvre pour nettoyer les puits de forage pétroliers forés au moyens de fluides à base d'huile, ainsi que les milieux aqueux détergents utilisés en détergence ménagère (lave-linge, lave-vaisselle, lavage de surfaces dures) ou en détergence industrielle et institutionnelle.

Lors de l'utilisation de milieux aqueux de dégraissage des tôles métalliques en milieu alcalin, la formation de mousse peut avoir comme origine la présence dans la composition de dégraissage d'agents tensioactifs détergents moussants, de même que la présence de savons issus des acides gras ou esters présents dans le milieu contaminant à éliminer.

Ces mêmes difficultés peuvent apparaître lors du dégraissage des plates-formes pétrolières. Il est à noter que dans le domaine lié à l'exploitation du pétrole, le moussage peut aussi être causé par la présence dans le pétrole brut, de composés moussants. Le phénomène de moussage est bien souvent dangereux dans cette application particulière. Ainsi, outre les problèmes évidents de sécurité existants sur des plates-formes rendues glissantes, la création

de mousse dans le puits lui-même, lors de l'opération de nettoyage de ce dernier avant la mise en production de ce dernier, a pour conséquence de diminuer la densité dans le puits (par inclusion d'air) et peut, par exemple, entraîner l'éruption incontrôlée du pétrole s'il s'agit d'un puits éruptif.

Lors de l'utilisation de milieux aqueux détergents en détergence ménagère ou en détergence industrielle et institutionnelle, l'apparition de mousse peut être due à la présence dans la composition détergente, d'agents tensioactifs détergents moussants, ainsi qu'à la présence de certaines salissures comme les protéines alimentaires du type albumine, lait ...

Ledit agent démoussant selon l'invention peut être mis en oeuvre dans le milieu aqueux dont il faut limiter le volume de mousse formé, soit par l'intermédiaire des compositions dégraissantes ou détergentes, par introduction dudit agent dans lesdites compositions au moment de la fabrication de celles-ci, ou par introduction dudit agent dans lesdites compositions au moment de l'utilisation de celles-ci, soit par addition directe dudit agent dans le milieu aqueux dont il faut limiter le volume de mousse formé.

Celui-ci est mis en oeuvre selon des quantités fonction de la quantité d'agent(s) moussant(s) présent(s) dans le milieu aqueux et du pouvoir moussant de ce ou ces dernier(s).

Dans les milieux aqueux de dégraissage en milieu alcalin des tôles métalliques, lesdits agents terpéniques polyalcoxylés démoussants peuvent être d'une manière générale présents à raison de l'ordre de 0,01 à 5g/l , de préférence de l'ordre de 0,1 à 1g/l dudit milieu.

Dans le domaine de l'exploitation pétrolière, et plus particulièrement du dégraissage des plates-formes, les milieux aqueux comprennent de l'ordre de 0,005 à 0,05g/l, de préférence de l'ordre de 0,015 à 0,025g/l dudit milieu (après dilution), en agents terpéniques polyalcoxylés démoussants.

Pour ce qui a plus particulièrement trait au nettoyage du puits, la quantité d'agent démoussant est mis en oeuvre avec une concentration comprise entre 0,5 et 10 % en poids dans la solution aqueuse, de préférence 1 à 5 %.

Dans le domaine de la détergence ménagère lave-linge, des quantités d'agents terpéniques polyalcoxylés démoussants de l'ordre de 2 à 10%, de préférence de l'ordre de 3 à 5% en poids par rapport à l'extrait sec du milieu lessiviel, pour une formulation détergente classique contenant de l'ordre de 5 à 15% d'agents tensioactifs anioniques et/ou non-ioniques, sont recommandées. En détergence ménagère lave-vaisselle, ces quantités peuvent être de l'ordre de 0,5 à 10%, de préférence de l'ordre de 1 à 3%.

Dans les milieux aqueux utilisés en détergence industrielle et institutionnelle lesdits agents terpéniques polyalcoxylés démoussants peuvent être d'une manière générale présents à raison de l'ordre de 0,005 à 0,05g/l, de préférence de l'ordre de 0,015 à 0,025g/l dudit milieu (après dilution).

Les milieux aqueux dégraissants ou détergents contenant ledit agent démoussant de l'invention, sont de préférence utilisés à une température au moins de l'ordre de leur température de trouble.

La présente invention a également pour objet les compositions dégraissantes ou détergentes comprenant au moins un composé terpénique polyalcoxylé de formule (I) tel que défini ci-dessus.

Les formulations de dégraissage des tôles métalliques en milieu alcalin, comprennent en outre :

- de l'ordre de 0 à 2%, généralement de 0,01 à 1% en poids (dans la solution aqueuse) d'au moins un agent tensioactif détergent anionique ou non-ionique tel que les alkyl($C_8$-$C_{16}$)benzène sulfonates, les alkyl($C_8$-$C_{20}$)sulfates, les alkylphénols éthoxylés, les alcools gras éthoxylés, les polymères séquencés d'oxyde d'éthylène et d'oxyde de propylène,
- de l'ordre de 5 à 20% en poids (dans la solution aqueuse) d'au moins un électrolyte hydrotrope tel que les benzènesulfonates, les mono- ou di-alkyl ($C_1$-$C_4$) benzènesulfonates, les toluène-, xylène- ou cumène-sulfonates,
- d'autres agents hydrotropes, tels que les alcools et les glycols,
- de l'ordre de 5 à 25% en poids (dans la solution aqueuse) d'au moins un agent séquestrant tel que l'acide nitriloacétique, l'acide éthylènediamine tétraacétique, l'acide éthylènediamine tétraméthylphosphonique, l'acide nitrilotriméthylène phosphonique ou leurs sels,
- des agents tampons tels que les alcanolamines, l'éthylènediamine...

Les compositions aqueuses mises en oeuvre pour le dégraissage des plates-formes pétrolières peuvent comprendre, outre le composé terpénique polyalcoxylé précité :

- de l'ordre de 0 à 2%, généralement de 0,01 à 1 % en poids (dans la solution aqueuse) d'au moins un agent tensioactif détergent anionique ou non-ionique tel que les alkyl($C_8$-$C_{16}$)benzène sulfonates, les alkyl($C_8$-$C_{20}$)sulfates, les alkylphénols éthoxylés, les alcools gras éthoxylés, les polymères séquencés d'oxyde d'éthylène et d'oxyde de propylène,
- de l'ordre de 5 à 20% en poids (dans la solution aqueuse) d'au moins un électrolyte hydrotrope tel que les benzènesulfonates, les mono- ou di-alkyl ($C_1$-$C_4$) benzènesulfonates, les toluène-, xylène- ou cumène-sulfonates,
- d'autres agents hydrotropes, tels que les alcools et les glycols,

- au moins un agent contrôlant le pH, tel que, par exemple, les carbonates, les sesquicarbonates, les bicarbonates de métaux alcalins,
- des additifs comme les enzymes en quantité pouvant aller jusqu'à 5 % du poids total du fluide aqueux, des agents inhibiteurs de corrosion des métaux.

Les milieux aqueux mis en oeuvre pour le enttoyage des puits de pétrole comprend, oure le composé terpénique polyalcoxylé :

- 3 à 40 % en poids du milieu aqueux d'agents tensio-actifs tels que :
agents tensio-actifs anioniques comme

  . les alkylesters sulfonates de formule R-CH($SO_3$M)-COOR', où R représente un radical alkyle en $C_{8-20}$, de préférence en $C_{10}$-$C_{16}$, R' un radical alkyle en $C_1$-$C_6$, de préférence en $C_1$-$C_3$ et M un cation alcalin (sodium, potassium, lithium), ammonium substitué ou non substitué (méthyl-, diméthyl-, triméthyl-, tetraméthylammonium, diméthylpiperidinium ...) ou dérivé d'une alcanolamine (monoéthanolamine, diéthanolamine, triéthanolamine ...). On peut citer tout particulièrement les méthyl ester sulfonates dont les radical R est en $C_{14}$-$C_{16}$ ;
  . les alkylsulfates de formule $ROSO_3M$, où R représente un radical alkyle ou hydroxyalkyle en $C_5$-$C_{24}$, de préférence en $C_{10}$-$C_{18,}$ M représentant un atome d'hydrogène ou un cation de même définition que ci-dessus, ainsi que leurs dérivés éthoxylénés (OE) et/ou propoxylénés (OP), présentant en moyenne de 0,5 à 30 motifs, de préférence de 0,5 à 10 motifs OE et/ou OP ;
  . les alkylamides sulfates de formule $RCONHR'OSO_3M$ où R représente un radical alkyle en $C_2$-$C_{22}$, de préférence en $C_6$-$C_{20}$, R' un radical alkyle en $C_2$-$C_3$, M représentant un atome d'hydrogène ou un cation de même définition que ci-dessus, ainsi que leurs dérivés éthoxylénés (OE) et/ou propoxylénés (OP), présentant en moyenne de 0,5 à 60 motifs OE et/ou OP ;
  . les sels d'acides gras saturés ou insaturés en $C_8$-$C_{24}$, de préférence en $C_{14}$-$C_{20}$, les alkylbenzènesulfonates en $C_9$-$C_{20}$, les alkylsulfonates primaires ou secondaires en $C_8$-$C_{22}$, les alkylglycérol sulfonates, les acides polycarboxyliques sulfonés décrits dans GB-A-1 082 179, les sulfonates de paraffine, les N-acyl N-alkyltaurates, les alkylphosphates, les iséthionates, les alkylsuccinamates les alkylsulfosuccinates, les monoesters ou diesters de sulfosuccinates, les N-acyl sarcosinates, les sulfates d'alkylglycosides, les polyéthoxycarboxylates
  le cation étant un métal alcalin (sodium, potassium, lithium), un reste ammonium substitué ou non substitué (méthyl-, diméthyl-, triméthyl-, tetraméthylammonium, diméthylpiperidinium ...) ou dérivé d'une alcanolamine (monoéthanolamine, diéthanolamine, triéthanolamine ...) ;

agents tensio-actifs non-ioniques comme

  . les alkylphénols polyoxyalkylénés (polyéthoxyéthylénés, polyoxypropylénés, polyoxybutylénés) dont le substituant alkyle est en $C_6$-$C_{12}$ et contenant de 5 à 25 motifs oxyalkylènes ; à titre d'exemple, on peut citer les TRITON X-45, X-114, X-100 ou X-102 commercialisés par Rohm & Haas Cy. ;
  . les glucosamide, glucamide, glycérolamide ;
  . les alcools aliphatiques en $C_8$-$C_{22}$ polyoxyalkylénés contenant de 1 à 25 motifs oxyalkylènes (oxyéthylène, oxypropylène) ; à titre d'exemple, on peut citer les TERGITOL 15-S-9, TERGITOL 24-L-6 NMW commercialisés par Union Carbide Corp., NEODOL 45-9, NEODOL 23-65, NEODOL 45-7, NEODOL 45-4 commercialisés par Shell Chemical Cy., KYRO EOB commercialisé par The Procter & Gamble Cy.
  . les produits résultant de la condensation de l'oxyde d'éthylène le composé résultant de la condensation de l'oxyde de propylène avec le propylène glycol, tels les PLURONIC commercialisés par BASF ;
  . les produits résultant de la condensation de l'oxyde d'éthylène le composé résultant de la condensation de l'oxyde de propylène avec l'éthylènediamine, tels les TETRONIC commercialisés par BASF ;
  . les oxydes d'amines tels que les oxydes d'alkyl $C_{10}$-$C_{18}$ diméthylamines, les oxydes d'alkoxy $C_8$-$C_{22}$ éthyl dihydroxy éthylamines ;
  . les alkylpolyglycosides décrits dans US-A-4 565 647 ;
  . les amides d'acides gras en $C_8$-$C_{20}$
  . les acides gras éthoxylés
  . les amides gras éthoxylés
  . les amines éthoxylées

agents tensio-actifs amphotères et zwitterioniques comme

- les alkyldiméthylbétaïnes, les alkylamidopropyldiméthylbétaïnes, les alkyltriméthylsulfobétaïnes, les produits de condensation d'acides gras et d'hydrolysats de proteines
- les alkylamphoacétates ou alkylamphodiacétates dont le groupe alkyle contient de 6 à 20 atomes de carbone.

- au moins un agent permettant d'ajuster le pH, tel que, par exemple, les carbonates, les sesquicarbonates, les bicarbonates de métaux alcalins, les hydroxydes de métaux alcalins ou alcalino-terreux,
- des additifs comme les enzymes en quantité pouvant aller jusqu'à 5 % du poids total du fluide aqueux, des agents inhibiteurs de corrosion des métaux,
- si nécessaire, des agents alourdissants de manière à maintenir une pression hydrostatique satisfaisante dans le puits :

A titre d'exemple de tels composés, on peut citer les sels solubles ou au moins partiellement solubles comme les halogénures de métaux alcalin ou alcalino-terreux, tels que le chlorure de sodium, le chlorure de potassium, le chlorure de césium, le chlorure de magnésium, le bromure de sodium, le bromure de potassium. On peut de même utiliser les sulfates, les carbonates, les bicarbonates, les silicates, les phosphates de métaux alcalins ou alcalino-terreux, seuls ou en mélange. Parmi les sels d'acides organiques, on peut mentionner tout spécialement les formiates de métaux alcalins ou alcalino-terreux, les acétates de métaux alcalins ou alcalino-terreux. Les halogénures, et plus particulièrement les chlorures de métaux alcalins ou alcalino-terreux sont préférés. Il est possible de mettre en oeuvre, bien que non préférés, des sels insolubles tels que les sulfates, silicates ou carbonates de métaux alcalino-terreux, comme le sulfate de baryum, le carbonate de calcium ; les bromures de métaux alcalino-terreux ou de zinc tels que le bromure de potassium, le bromure de zinc, etc.

- des hydrocolloïdes comme les polysaccharides d'origine végétale, tels que les polygalactomannanes et leur dérivés, comme le guar, l'hydroxypropylguar ; la cellulose et ses dérivés, les amidons et ses dérivés ; les polysaccharides d'origine bactérienne comme la gomme xanthane ou des dérivés désacétylés.

Les compositions détergentes pour lave-vaisselle comprennent généralement :

- au moins un agent tensio-actif en quantité pouvant aller de 0,5 à 10 %, de préférence de l'ordre de 1 à 5 %, du poids de ladite formulation détergente exprimé en matière sèche.

Parmi ceux-ci on peut citer :

- les agents tensio-actifs anioniques du type savons de métaux alcalins (sels alcalins d'acides gras en $C_8$-$C_{24}$), sulfonates alcalins (alcoylbenzène sulfonates en $C_8$-$C_{13}$, alcoylsulfonates en $C_{12}$-$C_{16}$), alcools gras en $C_6$-$C_{16}$ oxyéthylénés et sulfatés, alkylphénols en $C_8$-$C_{13}$ oxyéthylénés et sulfatés, les sulfosuccinates alcalins (alcoylsulfosuccinates en $C_{12}$-$C_{16}$)...
- les agents tensio-actifs non ioniques du type alcoylphénols en $C_6$-$C_{12}$ polyoxyéthylénés, alcools aliphatiques en $C_8$-$C_{22}$ polyoxyéthylénés et/ou polyoxypropylénés, les copolymères bloc oxyde d'éthylène - oxyde de propylène, les amides carboxyliques éventuellement polyoxyéthylénés ...,

Peuvent en outre être présents les additifs usuels entrant dans la composition des formulations détergentes pour le lavage en machine à laver la vaisselle.
Parmi ceux-ci on peut citer notamment :

- des "builders" (agents améliorant les propriétés de surface des tensio-actifs) du type :

  - phosphonates organiques comme ceux de la gamme DEQUEST® de MONSANTO à raison de 0 à 2% du poids total de formulation détergente exprimé en matière sèche,
  - acide nitriloacétique, acide N,N-dicarboxyméthyl-2-aminopentane dioïque, acide éthylènediamine tétraacétique, acide diéthylènetriamine pentaacétique, à raison de 0 à 10 % du poids total de formulation détergente exprimé en matière sèche,
  - acide citrique, acide gluconique ou acide tartrique ou leurs sels à raison de 0 à 10% du poids total de formulation détergente exprimé en matière sèche,

- des agents de blanchiment du type perborates, percarbonates associés ou non au N, N, N', N'-tétraacétyléthylènediamine (TAED) ou des produits chlorés du type des chloroisocyanurates à raison de 0 à 30 % du poids total de ladite formulation détergente exprimé en matière sèche,

- des agents auxiliaires de nettoyage du type copolymères d'acide acrylique et d'anhydride maléique ou des homopolymères d'acide acrylique à raison de 0 à 10 %,
- des silicates de métal alcalin de rapport molaire $SiO_2/Na_2O$ de l'ordre de 1 à 3,5 , comme agents anticorrosion des métaux, en quantité pouvant aller jusqu'à 50% environ du poids total de ladite formulation détergente exprimé en matière sèche,
- des agents alcalins comme les carbonates, bicarbonates, sesquicarbonates de métaux alcalins, les cogranulés de carbonate de métal alcalin et de silicate de métal alcalin de rapport molaire $SiO_2/Na_2O$ de l'ordre de 1,5 à 3,5 , avec un rapport pondéral carbonate /silicate de l'ordre de 5/95 à 45/55, contenant de l'eau selon un rapport pondéral eau/silicate exprimé en sec d'au moins 33/100, à raison de 0 à 50% du poids total de ladite formulation exprimé en matière sèche,
- des charges du type sulfate de sodium pour les détergents en poudre à raison de 0 à 50 % du poids total de ladite composition exprimé en matière sèche,
- d'autres additifs divers comme des enzymes en quantité pouvant aller jusqu'à 5 % du poids total de ladite formulation exprimé en matière sèche, parfums, colorants, agents inhibiteurs de corrosion des métaux, agents de mise en suspension des salissures ...

Les compositions détergentes pour lave-linge comprennent généralement :

- des agents tensioactifs, en quantités correspondant à environ 3-40% en poids par rapport à la composition détergente, choisis parmi les agents tensio-actifs anioniques, cationiques, non ioniques, zwittérioniques ou amphotères, seuls ou combinés, cités dans la description des fluides de nettoyage des puits. On pourra donc s'y reporter.
- des adjuvants améliiorant les propriétés des agents tensioactifs ("agents builders") en quantités correspondant à environ 5-50%, de preférence à environ 5-30% en poids pour les formules détergentes liquides, ou à environ 10-80%, de preférence 15-50% en poids pour les formules détergentes en poudres, agents builders tels que :
  <u>adjuvants ("builders") inorganiques comme</u>

  - les polyphosphates (tripolyphosphates, pyrophosphates, orthophosphates, hexamétaphosphates) de métaux alcalins, d'ammonium ou d'alcanolamines
  - les tetraborates ou les précurseurs de borates
  - les silicates, en particulier ceux présentant un rapport $SiO_2/Na_2O$ de l'ordre de 1,6/1 à 3,2/1 et les silicates lamellaires décrits dans US-A-4 664 839
  - les carbonates (bicarbonates, sesquicarbonates) alcalins ou alcalino-terreux
  - les cogranulés de silicates hydratés de métaux alcalins et de carbonates de métaux alcalins (sodium ou de potassium) riches en atomes de silicium sous forme Q2 ou Q3, décrits dans EP-A-488 868
  - les aminosilicates cristallins ou amorphes de métaux alcalins (sodium, potassium) ou d'ammonium, tels que les zéolithes A, P, X ... ; la zéolithe A de taille de particules de l'ordre de 0,1-10 micromètres est préférée

  <u>adjuvants ("builders") organiques comme</u>

  - les polyphosphonates hydrosolubles (éthane 1-hydroxy-1, 1-diphosphonates, sels de méthylène diphosphonates ...)
  - les sels hydrosolubles de polymères ou de copolymères carboxyliques ou leurs sels hydrosolubles tels que

    - les ethers polycarboxylates (acide oxydisuccinique et ses sels, tartrate monosuccinic acide et ses sels, tartrate disuccinic acide et ses sels
    - les ethers hydroxypolycarboxylates
    - l'acide citrique et ses sels, l'acide mellitique, l'acide succinique et leurs sels
    - les sels d'acides polyacétiques (éthylènediaminetetraacétates, nitrilotriacétates, N-(2 hydroxyéthyl)-nitrilodiacétates)
    - les acides alkyl C5-C20 succiniques et leurs sels( 2-dodécénylsuccinates, lauryl succinates, )
    - les esters polyacétals carboxyliques
    - l'acide polyaspartique, l'acide polyglutamique et leurs sels

  - les polyimides dérivés de la polycondensation de l'acide aspartique et/ou de l'acide glutamique
  - les dérivés polycarboxyméthylés de l'acide glutamique ou d'autres acides aminés

- des agents de blanchiment, en quantités d'environ 0,1-20%, de préférence environ 1-10% en poids, éventuellement associés à des activateurs de blanchiment, en quantités d'environ 0,1-60%, de préférence d'environ 0,5-40% en

poids, agents et activateurs tels que les
agents de blanchiment comme

- . les perborates tels que le perborate de sodium monohydraté ou tétrahydraté
- . les composés peroxygénés tels que le carbonate de sodium peroxyhydraté, le pyrophosphate peroxyhydraté, l'urée peroxyhydratée, le peroxyde de sodium, le persulfate de de sodium
de préférence associés à un activateur de blanchiment générant in situ dans le milieu lessiviel, un peroxyacide carboxylique ; parmi ces activateurs, on peut mentionner, la tetraacetyléthylène diamine, la tetraacétyl méthylène diamine, le tetraacétyl glycoluryl, le p-acétoxybenzène sulfonate de sodium, le pentaacétyl glucose, l'octaacétyl lactose ...
- . les acides percarboxyliques et leurs sels (appelés "percarbonates") tels que le monoperoxyphtalate de magnésium hexahydraté, le métachloroperbenzoate de magnésium, l'acide 4-nonylamino-4-oxoperoxybutyrique, l'acide 6-nonylamino-6-oxoperoxycaproique, l'acide diperoxydodécanedioique, le nonylamide de l'acide peroxysuccinique, l' acide décyldiperoxysuccinique.
Ces agents peuvent être associés à au moins un des agents anti-salissures ou anti-redéposition mentionnés ci-après.
Peuvent également être mentionnés des agents de blanchiment non oxygénés, agissant par photoactivation en présence d'oxygène, agents tels que les phtalocyanines d'aluminium et/ou de zinc sulfonées

- des agents antisalissures, en quantités d'environ 0,01-10%, de préférence environ 0,1-5%, et tout particulièrement de l'ordre de 0,2-3% en poids, agents tels que

  - . les dérivés cellulosiques tels que les hydroxyéthers de cellulose, la méthylcellulose, l'éthylcellulose, l'hydroxypropyl méthylcellulose, l'hydroxybutyl méthylcellulose
  - . les polyvinylesters greffés sur des troncs polyalkylenes tels que les polyvinylacétates greffés sur des troncs polyoxyéthylènes (EP-A-219 048)
  - . les alcools polyvinyliques
  - . les copolymères polyesters à base de motifs ethylène téréphtalate et/ou propylène téréphtalate et polyoxyéthylène téréphtalate, avec un rapport molaire (nombre de motifs) ethylène téréphtalate et/ou propylène téréphalate / (nombre de motifs) polyoxyéthylène téréphtalate de l'ordre de 1/10 à 10/1, de préférence de l'ordre de 1/1 à 9/1, les polyoxyéthylène téréphtalates présentant des unités polyoxyéthylène ayant un poids moléculaire de l'ordre de 300 à 5000, de préférence de l'ordre de 600 à 5000 (US-A-3 959 230, US-A-3 893 929, US-A-4 116 896, US-A-4 702 857, US-A-4 770 666) ;
  - . les oligomères polyesters sulfonés obtenus par sulfonation d'un oligomère dérivé de de l'alcool allylique éthoxylé, du diméthyltéréphtalate et du 1,2 propylène diol, présentant de 1 à 4 groupes sulfonés (US-A-4 968 451)
  - . les copolymères polyesters à base de motifs propylène téréphtalate et polyoxyéthylène téréphtalate et terminés par des motifs éthyles, méthyles (US-A-4 711 730) ou des oligomères polyesters terminés par des groupes alkylpolyéthoxy (US-A-4 702 857) ou des groupes anioniques sulfopolyéthoxy (US-A-4 721 580), sulfoaroyles (US-A-4 877 896)
  - . les copolymères polyesters sulfonés dérivés d'acide, anhydride ou diester téréphtalique, isophtalique et sulfoisophtalique et d'un diol (FR-A-2 720 399)

- des agents antiredéposition, en quantités d'environ 0,01-10% en poids pour une composition détergente en poudre, d'environ 0,01-5%en poids pour une composition détergente liquide, agents tels que

  - . les monoamines ou polyamines éthoxylées, les polymères d'amines éthoxylées (US-A-4 597 898, EP-A-11 984)
  - . la carboxyméthylcellulose
  - . les oligomères polyesters sulfonés obtenus par condensation de l'acide isophtalique, du sulfosuccinate de diméthyle et de diéthylène glycol (FR-A-2 236 926)
  - . les polyvinylpyrollidones

- des agents chélatants du fer et du magnésium, en quantités de l'ordre de 0,1-10%, de préférence de l'ordre de 0,1-3% en poids, agents tels que

  - . les aminocarboxylates tels que les éthylènediaminetétraacétates, hydroxyéthyl éthylènediaminetriacétates, nitrilotriacétates

- . les aminophosphonates tels que les nitrilotris(méthylène phosphonates)
- . les composés aromatiques polyfonctionnels tels que les dihydroxydisulfobenzènes
- . des agents dispersants polymériques, en quantité de l'ordre de 0,1-7% en poids,pour contrôler la dureté en calcium et magnésium, agents tels que
- . les sels hydrosolubles d'acides polycarboxyliques de masse moléculaire de l'ordre de 2000 à 100 000, obtenus par polymérisation ou copolymérisation d'acides carboxyliques éthyléniquement insaturés tels que acide acryliqiue, acide ou anhydride maleique, acide fumarique, acide itaconique, acide aconitique, acide mesaconique, acide citraconique, acide méthylènemalonique , et tout particulièrement les polyacrylates de masse moléculaire de l'ordre de 2 000 à 10 000 (US-A-3 308 067), les copolymères d'acide arylique et d'anhydride maleique de masse moléculaire de l'ordre de 5 000 à 75 000 (EP-A-66 915)
- . les polyéthylèneglycols de masse moléculaire de l'ordre de 1000 à 50 000

- des agents de fluorescence, en quantité d'environ 0,05-1,2% en poids, agents tels que les dérivés de stilbène, pyrazoline, coumarine, acide fumarique, acide cinnamique, , azoles, methinecyanines, thiophènes ... ("The production and application of fluorescent brightening agents" - M. Zahradnik, publié par John Wiley & Sons, New York-1982-)
- d'autres agents suppresseurs de mousses, en quantités pouvant aller jusqu'à 5% en poids, agents tels que

  - . les acides gras monocarboxyliques en $C_{10}$-$C_{24}$ ou leurs sels alcalins, d'ammonium ou alcanolamines, les triglycérides d'acides gras
  - . les hydrocarbures saturés ou insaturés aliphatiques, alicycliques, aromatiques ou hétérocycliques, tels que les paraffines, les cires
  - . les N-alkylaminotriazines
  - . les monostéarylphosphates, les monostéaryl alcool phosphates
  - . les huiles ou résines polyorganosiloxanes événtuellement combinées avec des particules de silice

- des agents adoucissants, en quantités d'environ 0,5-10% en poids, agents tels que les argiles
- des enzymes en quantité pouvant aller jusqu'à 5mg en poids, de préférence de l'ordre de 0,05-3mg d'enzyme active /g de composition détergente, enzymes telles que

  - . les protéases, amylases, lipases, cellulases, peroxydases (US-A-3 553 139, US-A-4 101 457, US-A-4 507 219, US-A-4 261 868

- d'autres additifs tels que

  - . des alcools (méthanol, éthanol, propanol, isopropanol, propanediol, éthylène glycol, glycérine)
  - . des agents tampons
  - . des parfums
  - . des pigments

Les compositions détergentes pour détergence industrielle et institutionnelle comprennent généralement des agents tensio-actifs non-ioniques (à raison de 0,05 à 50%, de préférence de 0,1 à 20% de la formulation), tels que

- . les alkylphénols polyoxyalkylénés (polyéthoxyéthylénés, polyoxypropylénés, polyoxybutylénés) dont le substituant alkyle est en $C_6$-$C_{12}$ et contenant de 5 à 25 motifs oxyalkylènes ; à titre d'exemple, on peut citer les TRITON X-45, X-114, X-100 ou X-102 commercialisés par Rohm & Haas Cy. ;
- . les glucosamides, glucamides, glycérolamides ;
- . les alcools aliphatiques en $C_8$-$C_{22}$ polyoxyalkylénés contenant de 1 à 25 motifs oxyalkylènes (oxyéthylène, oxypropylène) ; à titre d'exemple, on peut citer les TERGITOL 15-S-9, TERGITOL 24-L-6 NMW commercialisés par Union Carbide Corp., NEODOL 45-9, NEODOL 23-65, NEODOL 45-7, NEODOL 45-4 commercialisés par Shell Chemical Cy., les RHODASURF IDO60, RHODASURF LA90, RHODASURF IT070 commercialisés par RHONE-POULENC.
- . les oxydes d'amines tels que les oxydes d'alkyl $C_{10}$-$C_{18}$ diméthylamines, les oxydes d'alkoxy $C_8$-$C_{22}$ éthyl dihydroxy éthylamines ;
- . les alkylpolyglycosides décrits dans US-A-4 565 647 ;
- . les amides d'acides gras en $C_8$-$C_{20}$
- . les acides gras éthoxylés
- . les amines éthoxylées

des agents tensio-actifs amphotères et zwitterioniques (à raison de 0,05 à 50%, de préférence de 0,1 à 20% de la formulation), tels que

- les alkyldiméthylbétaïnes, les alkylamidopropyldiméthylbétaïnes, les alkyldiméthylsulfobétaïnes ou les alkylamidopropyldiméthylsulfobétaïnes comme le MIRATAINE CBS commercialisé par RHONE-POULENC, les produits de condensation d'acides gras et d'hydrolysats de proteines
- les alkylamphoacétates ou alkylamphodiacétates dont le groupe alkyle contient de 6 à 20 atomes de carbone. des agents tensio-actifs cationiques (à raison de 0,05 à 50%, de préférence de 0,1 à 20% de la formulation), tels que les sels d'alkylammonium de formule

$$R^1R^2R^3R^4N^+X^-$$

où
- $X^-$ représente un ion halogène, $CH_3SO_4^-$ ou $C_2H_5SO_4^-$
- $R^1$ et $R^2$ sont semblables ou différents et représentent un radical alkyl en $C_1$-$C_{20}$, un radical aryl ou benzyl
- $R^3$ et $R^4$ sont semblables ou différents et représentent un radical alkyl en $C_1$-$C_{20}$, un radical aryl ou benzyl ou un condensat oxyde d'éthylène et/ou de propylène $(CH_2CH_2O)_x$-$(CH_2CHCH_3O)_y$-H, où x et y vont de 0 à 30 et ne sont jamais nuls ensemble,

comme le RHODAQUAT TFR commercialisé par RHONE-POULENC.
des agents tensio-actifs anioniques (à raison de 0,05 à 50%, de préférence de 0,1 à 20% de la formulation), tels que

- les alkylesters sulfonates de formule R-CH($SO_3$M)-COOR', où R représente un radical alkyle en $C_{8-20}$, de préférence en $C_{10}$-$C_{16}$, R' un radical alkyle en $C_1$-$C_6$, de préférence en $C_1$-$C_3$ et M un cation alcalin (sodium, potassium, lithium), ammonium substitué ou non substitué (méthyl-, diméthyl-, triméthyl-, tetraméthylammonium, diméthylpiperidinium ...) ou dérivé d'une alcanolamine (monoéthanolamine, diéthanolamine, triéthanolamine ...). On peut citer tout particulièrement les méthyl ester sulfonates dont les radical R est en $C_{14}$-$C_{16}$ ;
- les alkylsulfates de formule $ROSO_3M$, où R représente un radical alkyle ou hydroxyalkyle en $C_5$-$C_{24}$, de préférence en $C_{10}$-$C_{18}$, M représentant un atome d'hydrogène ou un cation de même définition que ci-dessus, ainsi que leurs dérivés éthoxylénés (OE) et/ou propoxylénés (OP), présentant en moyenne de 0,5 à 30 motifs, de préférence de 0,5 à 10 motifs OE et/ou OP ;
- les alkylamides sulfates de formule $RCONHR'OSO_3M$ où R représente un radical alkyle en $C_2$-$C_{22}$, de préférence en $C_6$-$C_{20}$, R' un radical alkyle en $C_2$-$C_3$, M représentant un atome d'hydrogène ou un cation de même définition que ci-dessus, ainsi que leurs dérivés éthoxylénés (OE) et/ou propoxylénés (OP), présentant en moyenne de 0,5 à 60 motifs OE et/ou OP ;
- les sels d'acides gras saturés ou insaturés en $C_8$-$C_{24}$, de préférence en $C_{14}$-$C_{20}$, les alkylbenzènesulfonates en $C_9$-$C_{20}$, les alkylsulfonates primaires ou secondaires en $C_8$-$C_{22}$, les alkylglycérol sulfonates, les acides polycarboxyliques sulfonés décrits dans GB-A-1 082 179, les sulfonates de paraffine, les N-acyl N-alkyltaurates, les alkylphosphates, les iséthionates, les alkylsuccinamates les alkylsulfosuccinates, les monoesters ou diesters de sulfosuccinates, les N-acyl sarcosinates, les sulfates d'alkylglycosides, les polyéthoxycarboxylates
  le cation étant un métal alcalin (sodium, potassium, lithium), un reste ammonium substitué ou non substitué (méthyl-, diméthyl-, triméthyl-, tetraméthylammonium, diméthylpiperidinium ...) ou dérivé d'une alcanolamine (monoéthanolamine, diéthanolamine, triéthanolamine ...) ;
- les phosphates esters alkylés ou alkylarylés comme les RHODAFAC RA600, RHODAFAC PA15 ou RHODAFAC PA23 commercialisés par RHONE-POULENC.

des adjuvants de détergence ("builders") organiques (à raison de 0,1 à 50%, de préférence de 0,1 à 20% de la formulation), comme

- les polyphosphonates hydrosolubles
- les sels hydrosolubles de polymères ou de copolymères carboxyliques tels que
  - les ethers polycarboxylates ou hydroxypolycarboxylates
  - les citrates
  - les sels d'acides polyacétiques (éthylènediaminetetraacétates, nitrilotriacétates comme le NERVANAID NTA $Na_3$ commercialisé par RHONE-POULENC, N-(2 hydroxyéthyl)-nitrilodiacétates )
  - les sels d'acides alkyl $C_5$-$C_{20}$ succiniques

- les esters polyacétals carboxyliques
- les sels d'acides polyaspartiques ou polyglutamiques

<u>des adjuvants de détergence ("builders') minéraux</u> (à raison de 0,1 à 50%, de préférence de 0,1 à 20% de la formulation), comme

- les polyphosphates de métaux alcalins, d'ammonium ou d'alcanolamines tels que le RHODIAPHOS HPA3,5 commercialisé par RHONE-POULENC
- les pyrophosphates de métaux alcalins
- les silicates
- les carbonates alcalins ou alcalino-terreux
- les cogranulés de silicates hydratés de métaux alcalins et de carbonates de métaux alcalins (sodium ou de potassium) décrits dans EP-A-488 868, comme le NABION 15 commercialisé par RHONE-POULENC

<u>des agents hydrotropes</u> dans le cas des formules liquides, tels que le cumène ou xylène sulfonate de sodium, les phosphates esters comme le RHODAFAC HA70 commercialisé par RHONE-POULENC.

Les exemples suivants sont donnés à titre illustratif.

**TEST DE DEMOUSSAGE**

**<u>Principe du test</u>**

Le test consiste à observer pendant 15 minutes la mousse formée après 5 minutes d'agitation d'une solution à tester contenant un agent moussant et un agent démoussant et à comparer les résultats à ceux observés à l'absence d'agent démoussant (solution de référence).

**<u>Mode opératoire</u>**

On place 900g de solution à tester dans un bécher en inox de 2 litres (190 mm de hauteur et 120 mm de diamètre) préalablement nettoyé, calé par rapport à un système d'agitation constitué d'une pale (pale centripète Raynerie de 40 mm de diamètre, alimentée par un agitateur Raynerie de type Turbotest 1044) et d'une contrepale.
L'agitateur est mis en marche à 2000 tours/minute pendant 5 minutes.
Le bêcher est ensuite rétiré du système d'agitation ; son contenu est le plus rapidement possible versé dans une éprouvette graduée de 2 litres (NF B 35302) de façon à ce que le liquide et la mousse coulent le long de la paroi de l'éprouvette.
On déclenche le chronomètre et note le volume de mousse (situé entre le niveau haut et le niveau bas) après 0, 1, 2, 3, 4, 5, 10 et 15 minutes.
Ces mesures sont effectuées avec la solution de référence, puis avec la solution contenant l'agent démoussant à tester.

**<u>Expression des résultats</u>**

Ceux-ci peuvent être exprimés soit par le volume de mousse en ml observé, soit par mesure du paramètre d'efficacité E en % calculé comme suit :

$$E = [So - Sc]/So \times 100$$

So étant la surface de la courbe exprimant le volume de mousse formé avec la solution de référence en fonction du temps (0 à 15 minutes),
Sc étant la surface de la courbe exprimant le volume de mousse formé avec la solution testée en fonction du temps (0 à 15 minutes).
Plus Sc est faible, plus l'efficacité de l'agent démoussant est élevée.

**Exemple 1**

Préparation du NOPOL 3 OP / 6 OE / 15 OP

Dans un réacteur d'alcoxylation de 12 litres, on introduit du NOPOL de formule (II') (1 kg ; 6 moles) et une solution aqueuse d'hydroxyde de potassium (50%, 17,7g).

Le milieu réactionnel est déshydraté à 120°C sous courant d'azote.
On chauffe ensuite à 165°C et introduit de l'oxyde de propylène (1,045 kg, 3 équivalents molaires). A la fin de l'addition de l'oxyde de propylène, on introduit de l'oxyde d'éthylène (1,586 kg , 6 équivalents molaires).
A la fin de l'addition de l'oxyde d'éthylène, on introduit de l'oxyde de propylène (5,227 kg , 15 équivalents molaires).
Le milieu réactionnel est alors refroidi, neutralisé par addition d'acide acétique, jusqu'à l'obtention d'un pH de 7. Le liquide est filtré sur terre adsorbante (Clarcel DIC).
On obtient un liquide fluide limpide.
Une solution dans le butyldiglycol contenant 10% en poids dudit liquide ci-dessus préparé présente une température de trouble de 35,5°C.

**Exemple 2**

Préparation d'ARBANOL 2 OP / 7,5 OE / 5 OP

Dans un réacteur d'alcoxylation de 6 litres, on introduit de l'ARBANOL de formule (II") (1 kg ; 5 moles) et une solution aqueuse d'hydroxyde de potassium (50%, 9,4g).
Le milieu réactionnel est déshydraté à 120°C sous courant d'azote.
On chauffe ensuite à 165°C et introduit de l'oxyde de propylène (0,58 kg, 2 équivalents molaires). A la fin de l'addition de l'oxyde de propylène, on introduit de l'oxyde d'éthylène (1,652 kg , 7,5 équivalents molaires).
A la fin de l'addition de l'oxyde d'éthylène, on introduit de l'oxyde de propylène (1,452 kg , 5 équivalents molaires).
Le milieu réactionnel est alors refroidi, neutralisé par addition d'acide acétique, jusqu'à l'obtention d'un pH de 7. Le liquide est filtré sur terre adsorbante (Clarcel DIC).
On obtient un liquide fluide limpide.
Une solution dans l'eau distillée contenant 1 % en poids dudit liquide ci-dessus préparé présente une température de trouble de 32,5°C.

**Exemple 3**

Démoussage des Umilieux aqueux de dégraissage en milieu alcalin de surfaces métalliques (tôles, plates-formes)

- Test de démoussage -

Le test de démoussage décrit ci-dessus est réalisé à 50°C, sur un milieu aqueux constitué de

. 20g/l d'une lessive alcaline à 45% de matière active constituée de quantités pondérales égales de potasse, métasilicate de sodium (SIMET GA5, granulés de métasilicate anhydre et de métasilicate pentahydraté, commercialisé par Rhône-Poulenc) et de pyrophosphate tetrapotassique
. 1g/l d'IGEPAL NP10 (nonylphénol contenant 10 motifs oxyéthylène) moussant
. 0,5g/l d'agent tensioactif démoussant à tester

Les systèmes tensioactifs testés sont les suivants :

* 1 g/l d'IGEPAL NP10 seul (référence)
* 1 g/l d'IGEPAL NP10 + 0,5 g/l de NOPOL 3 OP / 6 OE / 15 OP préparé à l'exemple 1
* 1 g/l d'IGEPAL NP10 + 0,5 g/l de NOPOL 7,5 OE / 5,5 OP préparé comme décrit dans WO 96/01245
* 1 g/l d'IGEPAL NP10 + 0,5 g/l de NOPOL 2 OP / 5,1 OE préparé à l'exemple 2-2.6 de WO 96/01245
* 1 g/l d'IGEPAL NP10 + 0,5 g/l de PLURAFAC LF 431, agent démoussant du commerce (alcool x OE / y OP-$CH_3$ commercialisé par BASF)
* 1 g/l d'IGEPAL NP10 + 0,5 g/l de MIRAVON B12 DF, agent démoussant du commerce (alcool x OE / y OP commercialisé par Rhône-Poulenc)

Les températures de trouble des solutions alcalines obtenues sont les suivantes :

| solution alcaline contenant | Températures de trouble |
|---|---|
| 1 g/l d'IGEPAL NP10 seul | 61°C |
| 1 g/l d'IGEPAL NP10 + 0,5 g/l de NOPOL 3 OP / 6 OE / 15 OP | 42°C |

(suite)

| solution alcaline contenant | Températures de trouble |
|---|---|
| 1 g/l d'IGEPAL NP10 + 0,5 g/l de NOPOL 7,5 OE / 5,5 OP | 55°C |
| 1 g/l d'IGEPAL NP10 + 0,5 g/l de NOPOL 2 OP / 5,1 OE | 45°C |
| 1 g/l d'IGEPAL NP10 + 0,5 g/l de PLURAFAC LF 431 | 47°C |
| 1 g/l d'IGEPAL NP10 + 0,5 g/l de MIRAVON B12 DF | 41°C |

Les volumes de mousses mesurés en fonction du temps sont donnés dans le tableau ci-après :

| | volume de mousse en ml après | | | | | | | |
|---|---|---|---|---|---|---|---|---|
| système TA | 30" | 1 min | 2 min | 3 min | 4 min | 5 min | 10 min | 15 min |
| NP 10 seul | 1540 | 1280 | 1020 | 940 | 780 | 730 | 650 | 520 |
| NP 10 + NOPOL 3OP/6OE/15OP | 180 | 160 | 140 | 120 | 100 | 80 | 60 | 60 |
| NP 10 + PLURAFAC LF 431 | 500 | 400 | 240 | 170 | 130 | 110 | 80 | 60 |
| NP 10 + MIRAVON B12 DF | 160 | 140 | 90 | 80 | 70 | 70 | 60 | 50 |
| NP 10 + NOPOL 7,5 OE/5,5OP | 1600 | 1400 | 1200 | 1100 | 900 | 830 | 750 | 600 |
| NP 10 + NOPOL 2OP/5,1OE | 1500 | 1200 | 950 | 850 | 750 | 650 | 570 | 460 |

- Test de dégraissage -

- Graissage

Des plaques en acier prédégraissées de marque "Q-Panel" Stock n° R-36 type "Dull matt finish" de 0,8 x 76 x 152 mm sont immergées pendant 2 minutes dans une huile entière lubrifiante formulée pour laminage (de tôles en acier pour automobiles) de marque QUAKER 6130 N de Quaker Chemicals, puis suspendues pour égouttage pendant 24 heures.

- Nettoyage et rinçage

Les plaques graissées sont introduites dans une machine de dégraissage par aspersion CIEMME LARO 350. La température de dégraissage est de 45°C et la pression de 2 bar.
On prépare un milieu de dégraissage basique (pH 12-13) par dilution dans l'eau jusqu'à 8g/l d'une lessive aqueuse contenant 45% en poids de matière active constituée de quantités pondérales égales de potasse, métasilicate de sodium (SIMET GA5, granulés de métasilicate anhydre et de métasilicate pentahydraté, commercialisé par Rhône-Poulenc) et de pyrophosphate tetrapotassique et ajout de 1,5 g/l de système tensioactif à tester, soit

* 1,5 g/l d'IGEPAL NP10 seul
* 1,5 g/l d'un système constitué de 50% d'IGEPAL NP10 et de 50% de NOPOL 3 OP / 6 OE / 15 OP
* 1,5 g/l d'un système constitué de 50% d'IGEPAL NP10 et de 50% de MIRAVON B12 DF

Le temps d'immersion des plaques dans la solution de dégraissage est choisi pour permettre un dégraissage complet, c'est-à-dire correspondant, après rinçage des plaques sous filet d'eau courante pendant 5 secondes sur chaque face (débit = 2 l/min ; température 15-17°C), à la notation 4 (recouvrement complet des deux faces des plaques par un film d'eau continu).

- Résultats

Le tableau suivant indique le temps nécessaire pour arriver à un dégraissage total des plaques (notation 4).

| tensioactif | temps en secondes pour atteindre la notation 4 |
|---|---|
| IGEPAL NP10 | 30 |
| 50% IGEPAL NP10 + 50% NOPOL 3 OP / 6 OE / 15 OP | 30 |
| 50% IGEPAL NP10 + 50% MIRAVON B12 DF | 45 |

On constate que le NOPOL 3 OP / 6 OE / 15 OP selon l'invention, a permis de démousser parfaitement le milieu sans dégrader les performances de l'agent tensioactif dégraissant.

**Exemple 4**

Démoussage de milieux aqueux contenant de l'albumine (détergence ménagère en lave-vaisselle)

Le test de démoussage décrit ci-dessus est réalisé sur un milieu aqueux contenant

- de l'albumine (albumine d'oeuf en poudre de Prolabo - agent moussant) 0,66 g/l
- du carbonate de sodium 3,83 g/l
- agent démoussant à tester 0,33 g/l

Les agents tensioactifs testés sont

* NOPOL 3 OP / 6 OE / 15 OP préparé à l'exemple 1
* ARBANOL 2 OP / 7,5 OE / 5 OP préparé à l'exemple 2
* PLURAFAC LF 403 (alcool linéaire alcoxylé démoussant de BASF)
* MIRAVON B12 DF
* NOPOL 3 OP / 6 OE préparé comme décrit dans WO 96/01245 Les résultats obtenus sont les suivants :

| agent démoussant | efficacité E % |
|---|---|
| NOPOL 3 OP / 6 OE /15 OP | 99,1 |
| ARBANOL 2 OP / 7,5 OE / 5 OP | 81,4 |
| PLURAFAC LF 403 | 94,4 |
| MIRAVON B12 DF | 92,05 |
| NOPOL 3 OP / 6 OE | 66,59 |

**Exemple 5**

Démoussage de milieux aqueux contenant du lait (détergence industrielle et institutionnelle))

Le test de démoussage décrit ci-dessus est réalisé sur un milieu aqueux contenant

- de la poudre de lait écrémé à 0% de matière grasse
  ("spray" de lait de Bride - agent moussant) 25 g/l
- de la soude 20 g/l
- agent tensioactif démoussant à tester 0,5 g/l

Les agents tensioactifs testés sont

* NOPOL 3 OP / 6 OE / 15 OP
* ARBANOL 2 OP / 7,5 OE / 5 OP préparé à l'exemple 2
* TRITON DF16 (alcool linéaire polyéthoxylé démoussant de Union Carbide)
* MIRAVON B12 DF

Les résultats obtenus sont les suivants :

| agent démoussant | efficacité E % |
|---|---|
| NOPOL 3 OP / 6 OE / 15 OP | 95,5 |
| ARBANOL 2 OP / 7,5 OE /5 OP | 96,3 |
| TRITON DF16 | 60 |
| MIRAVON B12 DF | 97 |

**Exemple 6**

Le test de démoussage est réalisé en machine à laver le linge à hublot vertical de type AEG 2050, dans les conditions réelles d'un cycle de lavage à 95°C.
On utilise, à raison de 5g/l, une poudre de lavage contenant les composants suivants

| | |
|---|---|
| Glucopon 600 CS/UP/PF (tel quel) de Henkel (agent moussant) (polyglucoside présentant une chaîne linéaire en $C_{12}$-$C_{14}$, à 50-53% de matière active) | 12% |
| perborate monohydraté | 15% |
| TAED (solution aqueuse à 92% en poids de tetraacétylène diamine) | 5% |
| Nabion de Rhône-Poulenc (cogranulés de silicate de sodium et de carbonate de sodium) | 38% |
| carbonate de sodium | 8% |
| sulfate de sodium | 10,6% |
| Sokalan CP5 de BASF (copolymère acrylique/maleïque) | 5% |
| Dequest 2016 de Monsanto (phosphonate) | 1,6% |
| carboxyméthylcellulose | 1,5% |
| espérase | 0,15% |
| savinase | 0,15% |
| agent démoussant à tester | 3% |

Les agents tensioactifs testés sont les suivants

* NOPOL 3 OP / 6 OE / 15 OP
* MIRAVON B12 DF
* NOPOL 3 OP / 6 OE

On introduit par le hublot, une charge de lavage standardisée constituée de 10 torchons en coton et on lance le programme 5 de la machine à laver, de façon à travailler à humidité constante.
On introduit, à la fin de ce programme, 65g de poudre lavante et on lance le programme 95°C sans prélavage.
On mesure visuellement le volume de mousse formé au cours du programme.
Les résultats sont consignés sur le graphe de la figure 3, représentant la hauteur de mousse, exprimée en % de hauteur de hublot, observée en fonction du temps. Sur ce graphe figure également le profil de température de l'ensemble de l'opération.
Les résultats montrent que

* le NOPOL 3 OP / 6 OE / 15 OP démousse efficacement la formule lessivielle ; il est au moins aussi efficace que le MIRAVON B12 DF
* sans agent démoussant ou en présence de NOPOL 3 OP / 6 OE comme agent démoussant, la machine déborde, ce qui nécessite l'arrêt du test.

## Revendications

**1.** Composés terpéniques polyalcoxylés de formule (I)

$$Z - X - W - [CH(R^5)-CH(R^6)-O]_q - A \qquad (I)$$

formule dans laquelle

- le symbole Z représente un radical bicyclo[a,b,c]heptényle ou bicyclo[a,b,c]heptyle, éventuellement substitué par au moins un radical alkyle en $C_1$-$C_6$, méthyle de préférence,
  a, b et c étant tels que
  - * a + b + c = 5,
  - * a = 2, 3 ou 4
  - * b = 1 ou 2
  - * c = 0 ou 1
- le symbole X représente un groupement
  - * $-CH_2-C(R^1)(R^2)-O-$
  - * ou $-O-CH(R'^1)-CH(R'^2)-O-$

  les symboles $R^1$ et $R^2$ étant identiques ou différents et représentant un groupement (cyclo)alkyle ou (cyclo) alcényle linéaire ou ramifié en $C_1$-$C_6$, méthyle notamment, ou de préférence un atome d'hydrogène
  les symboles $R'^1$ et $R'^2$ étant identiques ou différents et représentant un groupement (cyclo)alkyle ou (cyclo) alcényle linéaire ou ramifié en $C_1$-$C_{22}$, méthyle notamment, ou de préférence un atome d'hydrogène
- les symboles $R^5$ et $R^6$ sont différents, l'un représentant un atome d'hydrogène et l'autre un groupement (cyclo) alkyle ou (cyclo)alcényle linéaire ou ramifié en $C_1$-$C_{22}$, méthyle de préférence
- q est une valeur moyenne pouvant aller de 1 à 30, de préférence de 5 à 20
- W représente un groupement polyséquencé constitué de séquences différentes - $[B]_n$ - et - $[C]_p$ -,
  - . B représentant un groupement $-CH(R^3)-CH(R^4)-O-$ , dans lequel $R^3$ et $R^4$ sont identiques ou différents et représentent un atome d'hydrogène ou un groupe en $C_1$-$C_{22}$ (cyclo)alkyle ou (cyclo)alcényle linéaire ou ramifié, de préférence méthyle, $R^3$ et $R^4$ étant différents lorsque l'un d'eux représente de l'hydrogène
  - . C représentant un groupement oxyéthyléné $-CH_2-CH_2-O-$ (EO)
  - . n étant une valeur moyenne pouvant aller de 1 à 10, de préférence de 2 à 4
  - . p étant une valeur moyenne pouvant aller de 1 à 100, de préférence de 3 à 20 ledit groupement polyséquencé W étant lié au motif X par une de ses séquences - $[B]_n$ - - A représente un atome d'hydrogène, un radical alkyle en $C_1$-$C_6$, un radical aryle ou alkylaryle, un atome d'halogène, un groupement $-CH_2-CH(OH)R^7$, où le symbole $R^7$ représente un radical alkyle linéaire ou ramifié ou cyclique en $C_1$-$C_{22}$ ou aryle, ou un groupement choisi parmi $-SO_3M$, $-OPO_3(M)_2$, $-(CH_2)a-COCM$, $-(CH_2)_b-SO_3M$, avec a et b allant de 1 à 6, M représentant H , Na , K , Li , N(RR'R"R"')+ où les symboles R, R', R" et R"' sont identiques ou différents et représentent un atome d'hydrogène ou un radical alkyle linéaire ou ramifié ou cyclique en $C_1$-$C_{22}$ éventuellement hydroxylé.

**2.** Composés terpéniques polyalcoxylés selon la revendication 1), **caractérisés en ce que** le symbole Z représente un radical $Z^1$ ou $Z^2$,

$Z^1$ étant un radical

- bicyclo[3.1.1]heptényle, de préférence substitué sur son atome de carbone en 6 par au moins un radical alkyle en $C_1$-$C_6$, tout particulièrement par deux radicaux méthyles, ledit radical bicyclo[3.1.1]heptényle étant lié au motif X de formule $-CH_2-C(R^1)(R^2)-O-$ , par l'intermédiaire de son atome de carbone en 2 ;
- ou bicyclo[2.2.1]heptényle, de préférence substitué sur son atome de carbone en 7 par au moins un radical alkyle en $C_1$-$C_6$, tout particulièrement par deux radicaux méthyles, ledit radical bicyclo[2.2.1]heptényle étant lié au motif X de formule $-CH_2-C(R^1)(R^2)-O-$, par l'intermédiaire de son atome de carbone en 2 ou

en 3 ;

et $Z^2$ étant un radical bicyclo[2.2.1]heptyle, de préférence substitué sur son atome de carbone en 7 par au moins un radical alkyle en $C_1$-$C_6$, tout particulièrement par deux radicaux méthyles,
ledit radical bicyclo[2.2.1]heptyle étant lié au motif X de formule -O-CH(R'1)-CH(R'2)-O- , par l'intermédiaire de son atome de carbone en 2 ou en 3.

**3.** Composés terpéniques polyalcoxylés selon la revendication 1) ou 2), **caractérisés en ce que** les dites séquences $[B]_n$ et $[CH(R^5)\text{-}CH(R^6)\text{-}O]_q$ sont des séquences polyoxypropylénées $[PO]_n$ et $[PO]_q$.

**4.** Composés terpéniques polyalcoxylés selon l'une quelconque des revendications 1) à 3), **caractérisés en ce que** le symbole W représente un groupement biséquencé $-[B]_n\text{-}[C]_p-$ , la séquence $[B]_n$ étant une séquence polyoxypropylénée $[PO]_n$ et la séquence $[C]_p$ étant une séquence polyoxyéthylénée $[EO]_p$.

**5.** Composés terpéniques polyalcoxylés selon la revendication 2), **caractérisés en ce qu'**ils ont pour formule

$$Z^1 - CH_2\text{-}CH_2\text{-} O - [PO]_n\text{-}[EO]_p\text{-} [PO]_q\text{-} A$$

ou

$$Z^2 - O -CH_2\text{-}CH_2\text{-} O - [PO]_n\text{-}[EO]_p\text{-} [PO]_q\text{-} A$$

**6.** Composés terpéniques polyalcoxylés selon l'une quelconque des revendications 1) à 5). **caractérisés en ce qu'**ils ont pour formule

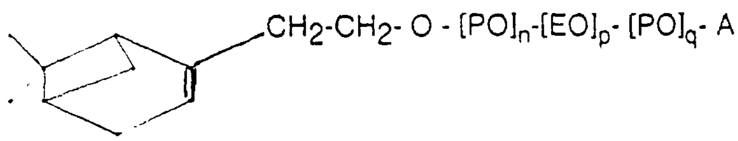

ou

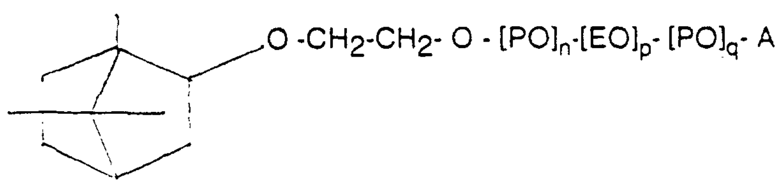

**7.** Composés terpéniques polyalcoxylés selon l'une quelconque des revendications 1) à 6), **caractérisés en ce que** les valeurs moyennes de n, p et q sont choisies de façon à ce qu'une solution à 1 % en poids dans l'eau distillée dudit composé présente un point de trouble inférieur à 40°C.

**8.** Procédé de préparation des composés terpéniques polyalcoxylés faisant l'objet de la revendication 1), par réactions de polyalcoxylations successives d'un réactif de formule Z - XH, où Z et X ont la définition donnée à la revendication 1), avec de l'oxyde d'alkylène (OA1) de formule

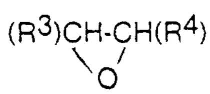

de l'oxyde d'alkylène (OE) de formule

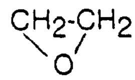

et de l'oxyde d'alkylène (OA2) de formule

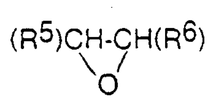

$R^3$, $R^4$, $R^5$ et $R^6$ ayant la définition donnée à la revendication 1), avec introduction successive des oxydes d'alkylènes (OA1) et (OE) et introduction finale d'oxyde d'alkylène (OA2), pour obtenir un produit de formule

$$Z - X - W - [CH(R^5)-CH(R^6)-O]_q\ H$$

dans laquelle W et q ont la définition donnée à la revendication 1), puis éventuellement fonctionnalisation pour transformer l'atome d'hydrogène terminal en un des substituants A autres que l'hydrogène tels que définis à la revendication 1).

**9.** Procédé selon la revendication 8), **caractérisé en ce que** le réactif Z - XH a pour formule

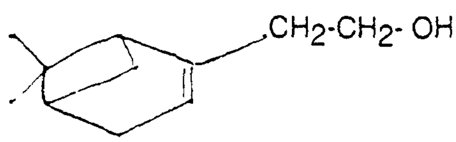

(II')

ou

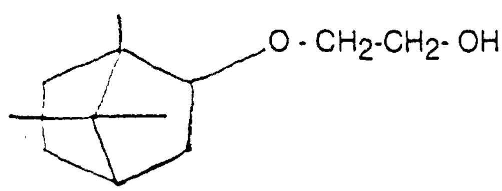

(II")

**10.** Procédé selon la revendication 8) ou 9), **caractérisé en ce que** les oxydes d'alkylènes (OA1) et (OA2) sont de l'oxyde de propylène.

**11.** Utilisation comme agent démoussant dans des milieux aqueux susceptibles de former des mousses, d'au moins un composé terpénique polyalcoxylé faisant l'objet de l'une quelconque des revendications 1) à 7) ou obtenu selon le procédé faisant l'objet de l'une quelconque des revendications 8) à 10).

**12.** Utilisation selon la revendication 11), **caractérisée en ce que** lesdits milieux aqueux, dont il faut limiter le volume de mousse susceptible de se former, sont les milieux aqueux de dégraissage en milieu alcalin des tôles métalliques, les milieux aqueux de degraissage des plates-formes de forage, les milieux aqueux mis en oeuvre pour nettoyer les puits de forage pétroliers forés au moyens de fluides à base d'huile, ainsi que les milieux aqueux détergents utilisés en détergence ménagère ou en détergence industrielle et institutionnelle.

**13.** Compositions aqueuses dégraissantes des surfaces métalliques en milieu alcalin, des puits de forages pétroliers, ou détergentes comprenant au moins un composé terpénique polyalcoxylé faisant l'objet de l'une quelconque des revendications 1) à 7) ou obtenu selon le procédé faisant l'objet de l'une quelconque des revendications 8) à 10).

## Patentansprüche

**1.** Polyalkoxyterpenoide der Formel (I)

$$Z\text{-}X\text{-}W\,[CH(R^5)\text{-}CH(R^6)\text{-}O]_q\text{-}A \qquad (I),$$

worin

- das Symbol Z einen Bicyclo[a,b,c]heptenyl- oder Bicyclo[a,b,c]heptyl-Rest darstellt, welcher gegebenenfalls durch wenigstens einen $C_1$-$C_6$-Alkylrest, vorzugsweise Methylrest, substituiert ist,
  wobei a, b und c die Bedeutung haben, dass

  * a + b + c = 5,
  * a = 2, 3 oder 4,
  * b = 1 oder 2,
  * c = 0 oder 1,

- das Symbol X eine Gruppe

  * $-CH_2-C(R^1)(R^2)-O-$
  * oder $-O-CH(R'^1)-CH(R'^2)-O-$

  darstellt, wobei die Symbole $R^1$ und $R^2$ identisch oder verschieden voneinander sind und eine lineare oder verzweigte $C_1$-$C_6$-(Cyclo)alkyl- oder (Cyclo)alkenylgruppe, insbesondere eine Methylgruppe oder vorzugsweise ein Wasserstoffatom darstellen,
  die Symbole $R'^1$ und $R'^2$ identisch oder verschieden voneinander sind und eine lineare oder verzweigte $C_1$-$C_{22}$-(Cyclo)alkyl- oder (Cyclo)alkenylgruppe, insbesondere eine Methylgruppe oder vorzugsweise ein Wasserstoffatom darstellen,
- die Symbole $R^5$ und $R^6$ verschieden voneinander sind, wobei eines ein Wasserstoffatom und das andere eine lineare oder verzweigte $C_1$-$C_{22}$-(Cyclo)alkyl- oder (Cyclo)alkenylgruppe, vorzugsweise eine Methylgruppe darstellt,
- q ein Mittelwert ist, der von 1 bis 30, vorzugsweise von 5 bis 20 reichen kann,
- W eine Polysequenzgruppe darstellt, die aus verschiedenen Sequenzen $-[B]_n-$ und $-[C]_p-$ besteht,

  . B eine $-CH(R^3)-CH(R^4)-O-$Gruppe darstellt, in welcher $R^3$ und $R^4$ identisch oder verschieden voneinander sind und ein Wasserstoffatom oder eine lineare oder verzweigte $C_1$-$C_{22}$-(Cyclo)alkyl- oder-(Cyclo)alkenylgruppe, vorzugsweise eine Methylgruppe darstellen, wobei $R^3$ und $R^4$ verschieden voneinander sind, wenn eine davon ein Wasserstoffatom darstellt,
  . wobei C eine Oxyethylengruppe $-CH_2-CH_2-O-$ (EO) darstellt,
  . n ein Mittelwert ist, welcher von 1 bis 10, vorzugsweise von 2 bis 4 reichen kann,
  . p ein Mittelwert ist, welcher von 1 bis 100, vorzugsweise von 3 bis 20 reichen kann,

  wobei die Polysequenzgruppe W mit der Grundeinheit X durch eine ihrer Sequenzen $-[B]_n-$ verbunden ist,
- A ein Wasserstoffatom, einen $C_1$-$C_6$-Alkylrest, einen Aryl- oder Alkylarylrest, ein Halogenatom, eine $-CH_2-CH(OH)R^7$-Gruppe darstellt, worin das Symbol $R^7$ einen linearen oder verzweigten oder cyclischen $C_1$-$C_{22}$-Alkyl- oder Arylrest darstellt oder eine Gruppe, die aus $-SO_3M$,
- $OPO_3(M)_2$, $-(CH_2)_a-COOM$, $-(CH_2)_b-SO_3M$ darstellt, wobei a und b 1 bis 6 bedeuten, M die Bedeutung H, Na, K, Li, $N(RR'R''R''')^+$ darstellen, worin die Symbole R, R', R'' und R''' identisch oder verschieden voneinander sind und ein Wasserstoffatom oder einen linearen oder verzweigten oder cyclischen, gegebenenfalls hydroxylierten $C_1$-$C_{22}$-Alkylrest darstellen.

**2.** Polyalkoxyterpenoide nach Anspruch 1, **dadurch gekennzeichnet, dass** das Symbol Z einen $Z^1$- oder $Z^2$-Rest darstellt, wobei
$Z^1$ ein Bicyclo[3.1.1]heptenylrest, der vorzugsweise an seinem Kohlenstoffatom 6 durch wenigstens einen $C_1$-$C_6$-Alkylrest, insbesondere durch zwei Methylreste substituiert ist,
wobei der Bicyclo[3.1.1]heptenylrest mit der Grundeinheit X der Formel $-CH_2-C(R^1)(R^2)-O-$ über sein Kohlenstoffa-

tom 2 verbunden ist;

- oder ein Bicyclo[2.2.1]heptenylrest ist, der vorzugsweise an seinem Kohlenstoffatom 7 durch wenigstens einen $C_1$-$C_6$-Alkylrest, vorzugsweise durch zwei Methylreste substituiert ist, wobei der Bicyclo[2.2.1]heptenylrest mit der Grundeinheit X der Formel $-CH_2-C(R^1)(R^2)-O-$ über sein Kohlenstoffatom 2 oder 3 verbunden ist; und
- $Z^2$ ein Bicyclo[2.2.1]heptylrest ist, der vorzugsweise an seinem Kohlenstoffatom 7 durch wenigstens einen $C_1$-$C_6$-Alkylrest, insbesondere durch zwei Methylreste substituiert ist, wobei der Bicyclo[2.2.1]heptylrest mit der Grundeinheit X der Formel $-O-CH(R'^1)-CH(R'^2)-O-$ durch sein Kohlenstoffatom 2 oder 3 verbunden ist.

3. Polyalkoxyterpenoide nach Anspruch 1 oder 2, **dadurch gekennzeichnet, dass** die Sequenzen $[B]_n$ und $[CH(R^5)-CH(R^6)-O]_q$ Polyoxypropylensequenzen $[PO]_n$ und $[PO]_q$ sind.

4. Polyalkoxyterpenoide nach einem der Ansprüche 1 bis 3, **dadurch gekennzeichnet, dass** das Symbol W eine Bise-quenzgruppe $-[B]_n-[C]_p-$ darstellt, wobei die Sequenz $[B]_n$ eine Polyoxypropylensequenz $[PO]_n$ und die Sequenz $[C]_p$ eine Polyoxyethylensequenz $[EO]_p$ ist.

5. Polyalkoxyterpenoide nach Anspruch 2, **dadurch gekennzeichnet, dass** sie die Formel

$$Z^1 - CH_2-CH_2- O -[PO]_n-[EO]_p-[PO]_q- A$$

oder

$$Z^2 - CH_2-CH_2- O -[PO]_n-[EO]_p-[PO]_q- A$$

besitzen.

6. Polyalkoxyterpenoide nach einem der Ansprüche 1 bis 5, **dadurch gekennzeichnet, dass** sie die Formel

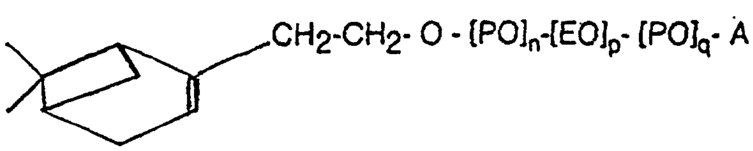

oder

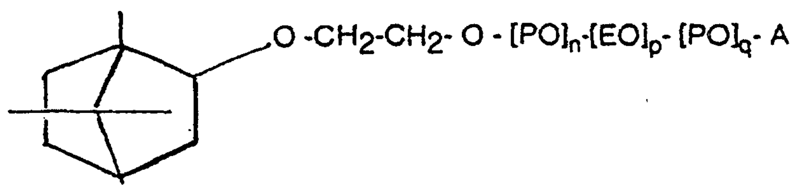

besitzen.

7. Polyalkoxyterpenoide nach einem der Ansprüche 1 bis 6, **dadurch gekennzeichnet, dass** die Mittelwerte von n, p und q derart ausgewählt sind, dass eine 1 Gew.%-ige Lösung in destilliertem Wasser der Verbindung einen Trübungspunkt von unter 40°C zeigt.

8. Verfahren zur Herstellung von Polyalkoxyterpenoide nach Anspruch 1, durch aufeinander folgende Polyalkoxylierungsreaktionen eines Reaktives der Formel Z - XH, worin Z und X die im Anspruch 1 angegebene Bedeutung besitzen, mit einem Alkylenoxid (OA1) der Formel

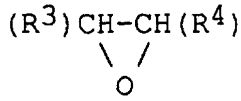 ,

einem Alkylenoxid (OE) der Formel

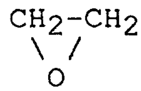 ,

und einem Alkylenoxid (OA2) der Formel

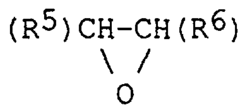 ,

worin $R^3$, $R^4$, $R^5$ und $R^6$ die im Anspruch 1 angegebene Bedeutung besitzen,
durch aufeinander folgendes Einführen der Alkylenoxide (OA1) und (OE) und zum Schluss dem Einführen des Alkylenoxides (OA2),
um ein Produkt der Formel

$$Z - X - W - [CH(R^5)-CH(R^6)-O]_q\ H$$

zu erhalten, worin W und q die im Anspruch 1 angegebene Bedeutung besitzen,
dann gegebenenfalls einer Funktionalisierung, um das endständige Wasserstoffatom in einen der Substituenten A, verschieden von Wasserstoff, wie in Anspruch 1 angegeben, umzuwandeln.

**9.** Verfahren nach Anspruch 8, **dadurch gekennzeichnet, dass** das Reaktiv Z - XH die Formel

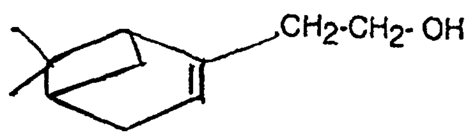 (II')

oder

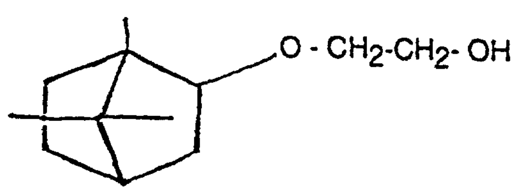 (II'')

besitzt.

**10.** Verfahren nach Anspruch 8 oder 9, **dadurch gekennzeichnet, dass** die Alkylenoxide (OA1) und (OA2) Propylenoxid sind.

**11.** Verwendung wenigstens eines Polyalkoxyterpenoides nach den Ansprüchen 1 bis 7 oder erhalten nach dem Verfahren nach einem der Ansprüche 8 bis 10 als Entschaumungsmittel in einem wässrigen Milieu, das der Schaumbildung unterworfen ist.

**12.** Verwendung nach Anspruch 11, **dadurch gekennzeichnet, dass** die wässrigen Milieus, deren Schaumvolumen, das sie bilden können, zu begrenzen ist, wässrige Milieus von alkalischen Entfettungsmilieus für Metallbleche, die wässrigen Milieus zur Entfettung von Bohrinseln, die wässrigen Milieus, welche zur Reinigung der Erdölbohrlöcher eingesetzt werden, die mittels eines öligen Fluides gebohrt werden sowie wässrige Waschhilfsmittel, welche im Haushalt, in der Industrie oder institutionell verwendet werden.

**13.** Wässrige Entfettungszusammensetzungen für metallische Oberflächen in alkalischem Milieu von Bohrlöchern der Erdölbohrung oder Detergentien, umfassend wenigstens ein Polyalkoxyterpeniod nach einem der Ansprüche 1 bis 7 oder erhalten nach dem Verfahren nach einem der Ansprüche 8 bis 10.

**Claims**

**1.** Polyalkoxylated terpenic compounds of formula (I)

$$Z\text{-}X\text{-}W\text{-}[CH(R^5)\text{-}CH(R^6)\text{-}O]_q\text{-}A \qquad (I)$$

in which formula:

- the Z symbol represents a bicyclo[a.b.c]heptenyl or bicyclo[a.b.c]heptyl radical, optionally substituted by at least one $C_1$-$C_6$ alkyl radical, preferably a methyl radical,
  a, b and c being such that:

  * a + b + c = 5,
  * a = 2, 3 or 4,
  * b = 1 or 2,
  * c = 0 or 1,

- the X symbol represents a group

  * $-CH_2-C(R^1)(R^2)-O-$,
  * or $-O-CH(R'^1)-CH(R'^2)-O-$,

  the $R^1$ and $R^2$ symbols being identical or different and representing a linear or branched $C_1$-$C_6$ (cyclo)alkyl or (cyclo)alkenyl group, in particular a methyl group, or preferably a hydrogen atom,
  the $R'^1$ and $R'^2$ symbols being identical or different and representing a linear or branched $C_1$-$C_{22}$ (cyclo)alkyl or (cyclo)alkenyl group, in particular a methyl group, or preferably a hydrogen atom,

- the $R^5$ and $R^6$ symbols are different, one representing a hydrogen atom and the other a linear or branched $C_1$-$C_{22}$ (cyclo)alkyl or (cyclo)alkenyl group, preferably a methyl group,
- q is a mean value which can range from 1 to 30, preferably from 5 to 20,
- W represents a polyblock group composed of different - $[B]_n$- and -$[C]_p$- blocks,

  · B representing a $-CH(R^3)-CH(R^4)-O-$ group, in which $R^3$ and $R^4$ are identical or different and represent a hydrogen atom or a linear or branched $C_1$-$C_{22}$ (cyclo)alkyl or (cyclo)alkenyl group, preferably a methyl group, $R^3$ and $R^4$ being different when one of them represents hydrogen,
  · C representing an oxyethylene group $-CH_2-CH_2-O-$ (EO),
  · n being a mean value which can range from 1 to 10, preferably from 2 to 4,
  · p being a mean value which can range from 1 to 100, preferably from 3 to 20,

the said polyblock group W being bonded to the X unit by one of its $-[B]_n-$ blocks,

- A represents a hydrogen atom, a $C_1$-$C_6$ alkyl radical, an aryl or alkylaryl radical, a halogen atom, a $-CH_2-CH(OH)R^7$ group, where the $R^7$ symbol represents a linear or branched or cyclic $C_1$-$C_{22}$ alkyl radical or an aryl radical, or a group chosen from $-SO_3M$, $-OPO_3(M)_2$, $-(CH_2)_a-COOM$ or $-(CH_2)_b-SO_3M$, with a and b ranging from 1 to 6 and M representing H, Na, K, Li or $N(RR'R''R''')^+$, where the R, R', R'' and R''' symbols are identical or different and represent a hydrogen atom or an optionally hydroxylated linear or branched or cyclic $C_1$-$C_{22}$ alkyl radical.

2. Polyalkoxylated terpenic compounds according to claim 1, **characterized in that** the Z symbol represents a $Z^1$ or $Z^2$ radical,

$Z^1$ being

- a bicyclo[3.1.1]heptenyl radical, preferably substituted on its carbon atom at the 6-position by at least one $C_1$-$C_6$ alkyl radical, very particularly by two methyl radicals, the said bicyclo[3.1.1]heptenyl radical being bonded to the X unit of formula $-CH_2-C(R^1)(R^2)-O-$ via its carbon atom at the 2-position;
- or a bicyclo[2.2.1]heptenyl radical, preferably substituted on its carbon atom at the 7-position by at least one $C_1$-$C_6$ alkyl radical, very particularly by two methyl radicals,

the said bicyclo[2.2.1]heptenyl radical being bonded to the X unit of formula $-CH_2-C(R^1)(R^2)-O-$ via its carbon atom at the 2-position or at the 3-position;
and $Z^2$ being a bicyclo[2.2.1]heptyl radical, preferably substituted on its carbon atom at the 7-position by at least one $C_1$-$C_6$ alkyl radical, very particularly by two methyl radicals,

the said bicyclo[2.2-1]heptyl radical being bonded to the X unit of formula $-O-CH(R'^1)-CH(R'^2)-O-$ via its carbon atom at the 2-position or at the 3-position.

3. Polyalkoxylated terpenic compounds according to claim 1 or 2, **characterized in that** the said $[B]_n$ and $[CH(R^5)-CH(R^6)-O]_q$ blocks are $[PO]_n$ and $[PO]_q$ polyoxypropylene blocks.

4. Polyalkoxylated terpenic compounds according to any one of claims 1 to 3, **characterized in that** the W symbol represents a $-[B]_n-[C]_p-$ diblock group, the $[B]_n$ block being a $[PO]_n$ polyoxypropylene block and the $[C]_p$ block being an $[EO]_p$ polyoxyethylene block.

5. Polyalkoxylated terpenic compounds according to claim 2, **characterized in that** they have the formula

$$Z^1-CH_2-CH_2-O-[PO]_n-[EO]_p-[PO]_q-A$$

or

$$Z^2-O-CH_2-CH_2-O-[PO]_n-[EO]_p-[PO]_q-A$$

6. Polyalkoxylated terpenic compounds according to any one of claims 1 to 5, **characterized in that** they have the formula

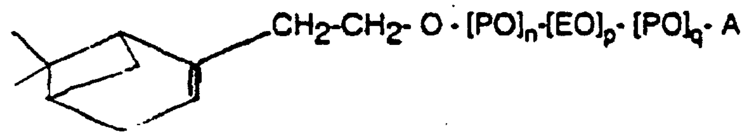

or

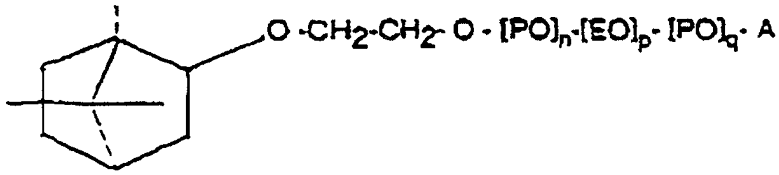

7. Polyalkoxylated terpenic compounds according to any one of claims 1 to 6, **characterized in that** the mean values of n, p and q are chosen so that a 1% by weight solution in distilled water of the said compound exhibits a cloud point of less than 40°C.

8. Process for the preparation of the polyalkoxylated terpenic compounds forming the subject-matter of claim 1 by successive polyalkoxylation reactions of a reactant of formula Z-XH, where Z and X have the definition given in claim 1, with alkylene oxide (AO1) of formula

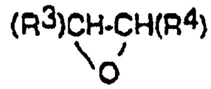

ethvlene oxide (EO) of formula

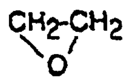

and alkylene oxide (AO2) of formula

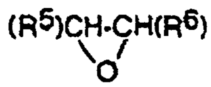

$R^3$, $R^4$, $R^5$ and $R^6$ having the definition given in claim 1, with successive introduction of the alkylene oxides (AO1) and (EO) and final introduction of alkylene oxide (AO2),
in order to obtain a product of formula

$$Z\text{-}X\text{-}W\text{-}[CH(R^5)\text{-}CH(R^6)\text{-}O]_qH$$

in which W and q have the definition given in claim 1, and then optionally functionalization in order to convert the terminal hydrogen atom into one of the A substituents, other than hydrogen, as defined in claim 1.

9. Process according to claim 8, **characterized in that** the Z-XH reactant has the formula

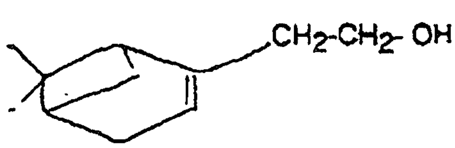

. (II')

or

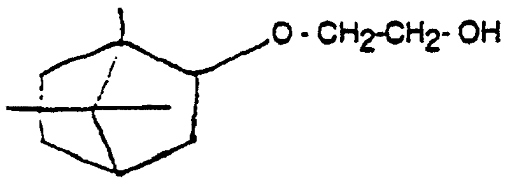

(II")

10. Process according to claim 8 or 9, **characterized in that** the alkylene oxides (AO1) and (AO2) are propylene oxide.

11. Use as defoaming agent in aqueous media which can form foams of at least one polyalkoxylated terpenic compound forming the subject-matter of any one of claims 1 to 7 or obtained according to the process forming the subject-matter of any one of claims 8 to 10.

12. Use according to claim 11, **characterized in that** the said aqueous media, for which it is necessary to limit the volume of foam which can be formed, are aqueous media for degreasing metal sheets in alkaline medium, aqueous media for degreasing drilling platforms, aqueous media employed for cleaning oil drilling wells drilled by means of oil-based fluids, and aqueous detergent media used in household detergency or in industrial and institutional detergency.

13. Aqueous compositions for degreasing metal surfaces in alkaline medium or oil drilling wells or detergents comprising at least one polyalkoxylated terpenic compound forming the subject-matter of any one of claims 1 to 7 or obtained according to the process forming the subject-matter of any one of claims 8 to 10.

**FIGURE 1**

figure 1.a

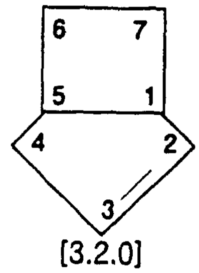

[3.2.0]

figure 1.b

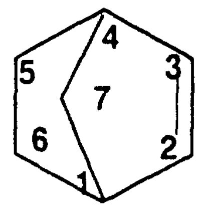

[2.2.1]

figure 1.c

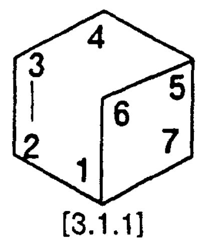

[3.1.1]

figure 1.d

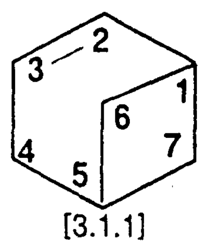

[3.1.1]

figure 1.e

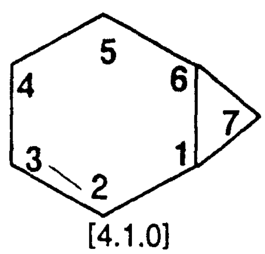

[4.1.0]

figure 1.f

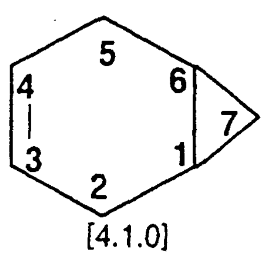

[4.1.0]

FIGURE 2

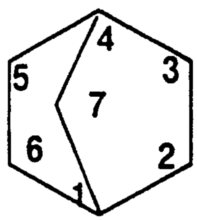

[2.2.1]

FIGURE 3

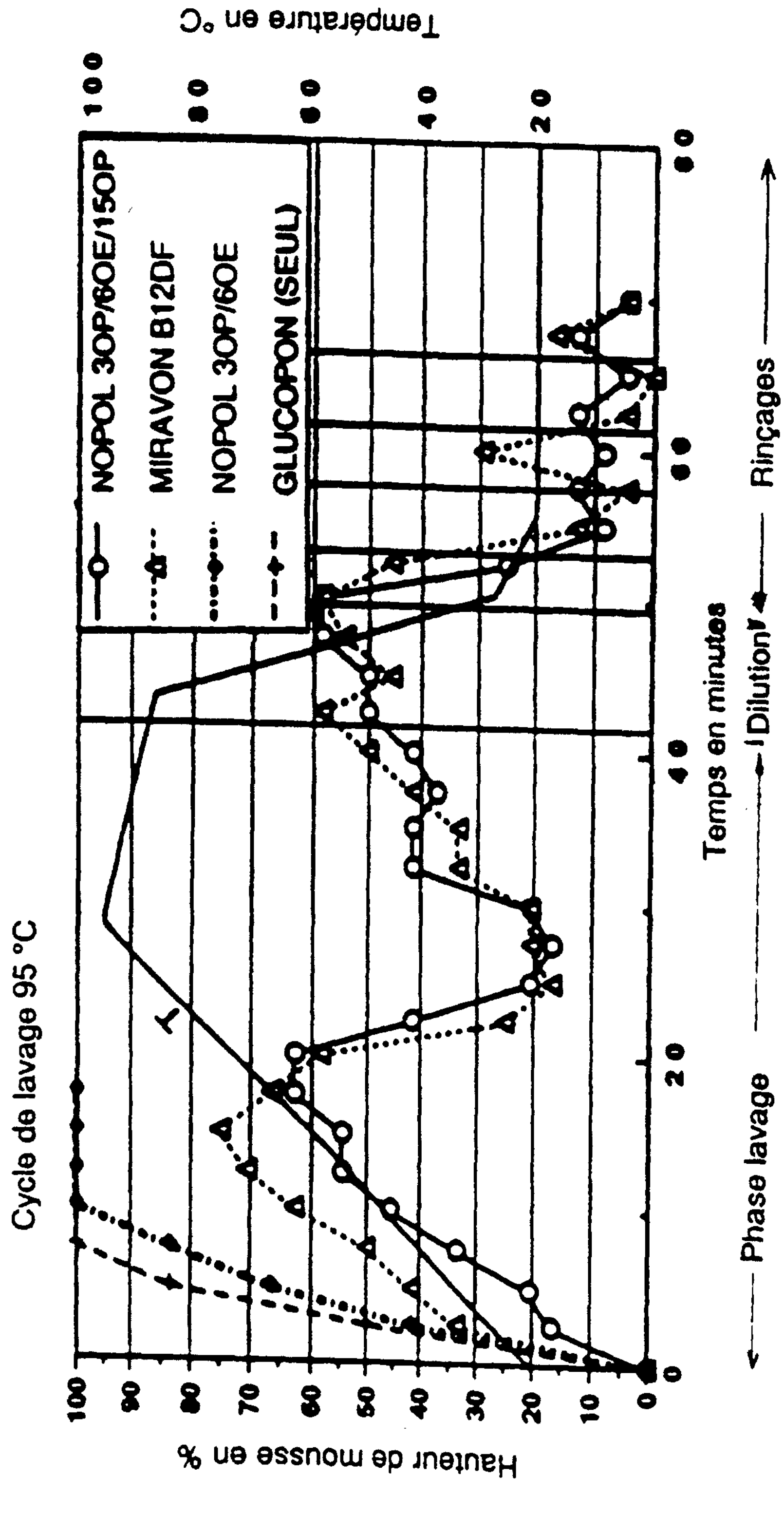
Cycle de lavage 95 °C
NOPOL 3OP/6OE/15OP
MIRAVON B12DF
NOPOL 3OP/6OE
GLUCOPON (SEUL)
Température en °C
Hauteur de mousse en %
Temps en minutes
Phase lavage
Dilution
Rinçages
100
90
80
70
60
50
40
30
20
10
0